# EUROPEAN PATENT SPECIFICATION

(11) **EP 2 021 803 B1**
(45) Date of publication and mention of the grant of the patent: **08.02.2012**
(21) Application number: 07840189.0
(22) Date of filing: 18.05.2007
(51) Int. Cl.: G01N 33/574

(54) **ASSESSING OUTCOMES FOR BREAST CANCER PATIENTS**
BEURTEILUNG DER ERGEBNISSE FÜR BRUSTKREBSPATIENTEN
ÉVALUATION DE RÉSULTATS POUR PATIENTES ATTEINTS DU CANCER DU SEIN

(30) Priority: 18.05.2006 US 801496 P
(43) Date of publication of application: 11.02.2009
(73) Proprietor: Mayo Foundation for Medical Education and Research, Rochester, MN 55905 (US)
(72) Inventor: GOETZ, Matthew, P., Rochester, Minnesota 55906 (US); INGLE, James, N., Rochester, Minnesota 55902 (US); COUCH, Fergus, J., Rochester, Minnesota 55902 (US); AMES, Matthew, M., Rochester, Minnesota 55902 (US)
(74) Representative: Grund, Martin
(86) International application number: PCT/US2007/069305
(87) International publication number: WO 2008/143669

(56) References cited:
- WO-A-2005/028681
- WO-A1-2006/004600
- US-A- 2003 170 176
- GOETZ M P ET AL: "An index based on HOXB13/IL17BR and CYP2D6 for determination of relapse and survival in tamoxifen-treated node negative breast cancer." BREAST CANCER RESEARCH AND TREATMENT, vol. 100, no. Suppl. 1, 2006, page S53, XP002572496 & 29TH ANNUAL SAN ANTONIO BREAST CANCER SYMPOSIUM; SAN ANTONIO, TX, USA; DECEMBER 14 -17, 2006 ISSN: 0167-6806
- GOETZ MATTHEW P ET AL: "A two-gene expression ratio of homeobox 13 and interleukin-17B receptor for prediction of recurrence and survival in women receiving adjuvant tamoxifen." CLINICAL CANCER RESEARCH : AN OFFICIAL JOURNAL OF THE AMERICAN ASSOCIATION FOR CANCER RESEARCH 1 APR 2006, vol. 12, no. 7 Pt 1, 1 April 2006 (2006-04-01), pages 2080-2087, XP002572497 ISSN: 1078-0432
- GOETZ M P ET AL: "Pharmacogenetics of tamoxifen biotransformation is associated with clinical outcomes of efficacy and hot flashes" JOURNAL OF CLINICAL ONCOLOGY 2005 US, vol. 23, no. 36, 2005, pages 9312-9318, XP002572498 ISSN: 0732-183X
- MA X.J. ET AL.: 'A two-gene expression ratio predicts clinical outcome in breast cancer patients treated with tamoxifen' CANCER CELL vol. 5, June 2004, pages 607 - 616, XP002317299
- MA X.-J. ET AL.: 'The HOXB13:IL17BR expression index is a prognostic factor in early-stage breast cancer' J. CLIN. ONCOL. vol. 24, no. 28, 01 October 2006, pages 4611 - 4619, XP008101969
- JIN Y. ET AL.: 'CYP2D6 Genotype, Antidepressant Use, and Tamoxifen Metabolism During Adjuvant Breast Cancer Treatment' J. NATL. CANCER INST. vol. 97, 2005, pages 30 - 39, XP008101348
- WEGMAN P. ET AL.: 'Genotype of metabolic enzymes and the benefit of tamoxifen in postmenopausal breast cancer patients' BREAST CANCER RESEARCH vol. 7, 2005, pages R284 - R290, XP021012113
- BORGES S. ET AL.: 'Quantitative effect of CYP2D6 genotype and inhibitors on tamoxifen metabolism: implication for optimization of breast cancer treatment' CLIN. PHARMACOL. THER. vol. 80, no. 1, July 2006, pages 61 - 74, XP005523877

### SEQUENCE LISTING

<110> Mayo Foundation for Medical Education and Research
<120> Assessing Outcomes for Breast Cancer Patients
<130> 07039-696WO1
<140> PCTUS0769305
   <141> 2007-05-25
<150> US 60/801,496
   <151> 2006-05-18
<160> 6
<170> FastSEQ for windows version 4.0
<210> 1
   <211> 23
   <212> RNA
   <213> Artificial Sequence
<220>
   <223> oligonucleotide
<400> 1
   gccatgatcg ttagcctcat att 23
<210> 2
   <211> 25
   <212> DNA
   <213> Artificial Sequence
<220>
   <223> oligonucleotide
<400> 2
   caattcatga aagcggtttc taaag 25
<210> 3
   <211> 22
   <212> DNA
   <213> Artificial Sequence
<220>
   <223> oligonucleotide
<400> 3
   tctatctaga gctctgtaga gc 22
<210> 4
   <211> 20
   <212> DNA
   <213> Artificial Sequence
<220>
   <223> oligonucleotide
<400> 4
   ggcttcctat cccaccaatt 20
<210> 5
   <211> 19
   <212> DNA
   <213> Artificial sequence
<220>
   <223> oligonucleotide
<400> 5
   aggctgtttg taggctgca 19
<210> 6
   <211> 20
   <212> DNA
   <213> Artificial Sequence
<220>
   <223> oligonucleotide
<400> 6
   cagggaaaaa acgtgtgatg 20

## Description

### BACKGROUND

### 1. Technical Field

This document relates to methods and materials involved in assessing the outcome of cancer patients.

### 2. Background Information

In the adjuvant treatment of estrogen receptor (ER) positive breast cancer, hormonal therapy reduces the risk of breast cancer recurrence and decreases mortality. Tamoxifen, one of the most commonly used medications in the adjuvant treatment of ER positive breast cancer, is a selective ER modulator that competes with estrogen for binding to the ER. When administered to women with surgically treated ER positive breast cancer, tamoxifen reduces the risk of recurrence and death when taken for five years.

The ER and progesterone receptor are the most important tumor markers that predict response to tamoxifen (Bardou et al., J. Clin. Oncol., 21(10):1973-9 (2003)). Because a significant proportion of ER positive breast cancers fail to respond or eventually develop resistance to tamoxifen, additional prognostic markers including tumor size, tumor grade, and nodal status are commonly used by physicians to make treatment decisions. Clinical studies have demonstrated, however, that even in "good prognosis" tumors (e.g., estrogen positive, lymph node negative), up to 20% of women will experience recurrence despite 5 years of adjuvant tamoxifen therapy (Fisher et al., J. Natl. Cancer Inst., 89(22):1673-82 (1997) and Fisher et al., Lancet, 364(9437):858-68 (2004)). These findings indicate the need for additional markers that will identify women at high risk for recurrence.

Goetz et al., Clinical Cancer Research, 12(7): 2080-2087 (2006) describe a two-gene expression ratio of Homeobox 13 and the Interleukin-17B receptor for the prediction of recurrence and survival in women receiving adjuvant tamoxifen. Goetz et al., Journal of Clinical Oncology, 23 (36): 9312-9318 (2005) deal with the pharmacogenetics of tamoxifen biotransformation and its association with clinical outcomes of efficacy and hot flashes.

### SUMMARY

The invention can be defined by the appendent claims.

According to one embodiment of the invention, a method for assessing the likelihood of cancer relapse is provided, wherein said method comprises:
determining whether or not a breast cancer patient being treated with tamoxifen contains cancer tissue with a HOXB13:IL17BR expression ratio greater than -1.339,
determining whether or not said breast cancer patient contains a CYP2D6 *4/WT or a CYP2D6 *4/*4 genotype, and
determining whether or not said breast cancer patient is taking medication selected from the group consisting of fluoxetine, paroxetine sertraline, cimetidine, amiodarone, doxepin, ticlopidine, and haloperidol,
wherein the presence of said HOXB13:IL17BR expression ratio greater than -1.339, the presence of said genotype and taking said medication indicates that said patient is likely to experience cancer relapse.

According to one embodiment of the invention, a method for assessing the likelihood of cancer survival is provided, wherein said method comprises:
determining whether or not a breast cancer patient being treated with tamoxifen contains cancer tissue with a HOXB13:IL17BR expression ratio less than -1.339,
determining whether or not said breast cancer patient contains a CYP2D6 *4/WT or a CYP2D6 *4/*4 genotype, and
determining whether or not said breast cancer patient is taking medication selected from the group consisting of fluoxetine, paroxetine sertraline, cimetidine, amiodarone, doxepin, ticlopidine, and haloperidol,
wherein the presence of said HOXB13:IL17BR expression ratio less than -1.339, the absence of said genotype and not taking said medication indicates that said patient is likely to experience longer survival than a comparable breast cancer patient who contains cancer tissue with a HOXB13:IL17BR expression ratio greater than -1.339 who contains said genotype and who is taking said medication.

According to one embodiment of the invention, a method for assessing the likelihood of cancer relapse is provided, wherein said method comprises:
determining whether or not a breast cancer patient taking tamoxifen contains cancer tissue with a HOXB13:IL17BR expression ratio greater than -1.339, and
determining whether or not said breast cancer patient is taking medication selected from the group consisting of fluoxetine, paroxetine sertraline, cimetidine, amiodarone, doxepin, ticlopidine, and haloperidol, wherein said medication is being taken concurrently with said tamoxifen, wherein the presence of said HOXB13:IL17BR expression ratio greater than -1.339 and taking said medication indicates that said patient is likely to experience cancer relapse.

This document provides methods and materials related to assessing the likely outcome for mammals (e.g., humans) with cancer (e.g., breast cancer). For example, this document provides methods and materials that involve assessing a patient's (1) ratio of HOXB13 polypeptide expression to IL-17BR polypeptide expression, (2) cytochrome P450, family 2, subfamily D, polypeptide 6 (CYP2D6) genotype, (3) medication history, or (4) a combination thereof, to determine the likelihood of a breast cancer patient to experience breast cancer relapse or death. As described herein, a high HOXB 13 to IL-17BR expression ratio is associated with increased relapse and death in patients with resected node negative, ER positive breast cancer treated with tamoxifen and may identify patients in whom alternative therapies should be studied. Patients with the CYP2D6 *4/*4 genotype tend to have a higher risk of disease relapse and death as well as a lower incidence of hot flashes, relative to patients heterozygous or homozygous for the wild-type CYP2D6 allele. Patients taking fluoxetine, paroxetine sertraline, cimetidine, amiodarone, doxepin, ticlopidine, or haloperidol medication while being treated with tamoxifen also tend to have a higher risk of disease relapse and death as well as a lower incidence of hot flashes, relative to patients who are not taking those medications and who are heterozygous or homozygous for the wild-type CYP2D6 allele.

In some cases, the likely outcome for a mammal (e.g., a human) with cancer (e.g., breast cancer) can be determined by assessing the mammal's (1) ratio of HOXB13 polypeptide expression to IL-17BR polypeptide expression and (2) CYP2D6 genotype. In some cases, the likely outcome for a mammal (e.g., a human) with cancer (e.g., breast cancer) can be determined by assessing the mammal's (1) ratio of HOXB13 polypeptide expression to IL-17BR polypeptide expression and (2) medication history. In some cases, the likely outcome for a mammal (e.g., a human) with cancer (e.g., breast cancer) can be determined by assessing the mammal's (1) ratio of HOXB13 polypeptide expression to IL-17BR polypeptide expression, (2) CYP2D6 genotype, and (3) medication history.

In general, one aspect of this document features a method for assessing the likelihood of cancer relapse. The method comprises, or consists essentially of, determining whether or not a node-negative breast cancer patient contains cancer tissue with a HOXB13:IL17BR expression ratio greater than -1.339, wherein the presence of the HOXB113:IL17BR expression ratio greater than - 1.339 indicates that the patient is likely to experience cancer relapse, and wherein the absence of the HOXB13:IL17BR expression ratio greater than - 1.339 indicates that the patient is likely to experience a relapse-free survival or a disease-free survival.

This document also features a method for assessing the likelihood of cancer survival. The method comprises, or consists essentially of, determining whether or not a node-negative breast cancer patient contains cancer tissue with a HOXB13:IL17BR expression ratio less than -1.339, wherein the presence of the HOXB13:IL17BR expression ratio less than -1.339 indicates that the patient is likely to experience longer survival than a comparable node-negative breast cancer patient who contains cancer tissue with a HOXB13:IL17BR expression ratio greater than -1.339.

This document also features a method for assessing the likelihood of cancer relapse. The method comprises, or consists essentially of, determining whether or not a breast cancer patient contains a CYP2D6 *4/WT or a CYP2D6 *4/*4 genotype, where the presence of the genotype indicates that the patient is likely to experience cancer relapse, and where the absence of the genotype indicates that the patient is likely to experience a relapse-free survival or disease-free survival.

This document also features a method for assessing the likelihood of a breast cancer patient to have hot flashes when treated with tamoxifen. The method comprises, or consists essentially of, determining whether or not the breast cancer patient contains a CYP2D6 *4/*4 genotype, where the presence of the genotype indicates that the patient is likely to experience hot flashes when treated with tamoxifen, and where the absence of the genotype indicates that the patient is not likely to experience hot flashes when treated with tamoxifen.

This document also features a method for assessing the likelihood of cancer relapse. The method comprises, or consists essentially of, (1) determining whether or not a breast cancer patient (e.g., a node-negative breast cancer patient) contains cancer tissue with a HOXB13:IL17BR expression ratio greater than -1.339, and (2) determining whether or not the breast cancer patient contains a CYP2D6 *4/WT or a CYP2D6 *4/*4 genotype, wherein the presence of the HOXB13:IL17BR expression ratio greater than -1.339 or the presence of the genotype indicates that the patient is likely to experience cancer relapse, and wherein the absence of the HOXB13:IL17BR expression ratio greater than -1.339 and the absence of the genotype indicates that the patient is likely to experience a relapse-free survival or a disease-free survival. The method also can include determining whether or not the breast cancer patient has taken a drug that inhibits CYP2D6 (e.g., fluoxetine, paroxetine sertraline, cimetidine, amiodarone, doxepin, ticlopidine, and haloperidol). A patient that has taken a drug that inhibits CYP2D6 is more likely to experience cancer relapse than a patient that has not taken a drug that inhibits CYP2D6. Determining whether or not the breast cancer patient contains cancer tissue with the HOXB13:IL17BR expression ratio greater than -1.339 can comprise using the HOXB13:IL-17BR calculation method.

This document also features a method for assessing the likelihood of cancer survival. The method comprises, or consists essentially of, (1) determining whether or not a breast cancer patient (e.g., a node-negative breast cancer patient) contains cancer tissue with a HOXB13:IL17BR expression ratio less than -1.339, and (2) determining whether or not the breast cancer patient contains a CYP2D6 *4/WT or a CYP2D6 *4/*4 genotype, wherein the presence of the HOXB13:IL17BR expression ratio less than -1.339 or the absence of the genotype indicates that the patient is likely to experience longer survival than a comparable node-negative breast cancer patient who contains cancer tissue with a HOXB13:IL17BR expression ratio greater than -1.339 and who has the genotype. Determining whether or not the breast cancer patient contains cancer tissue with the HOXB13:IL17BR expression ratio less than - 1.339 can comprise using the HOXB13:IL-17BR calculation method.

This document also features a method for assessing the likelihood of cancer relapse. The method comprises, or consists essentially of, (a) determining whether or not a breast cancer patient taking tamoxifen contains cancer tissue with a HOXB13:IL17BR expression ratio greater than - 1.339, and (b) determining whether or not the breast cancer patient is taking medication selected from the group consisting of fluoxetine, paroxetine sertraline, cimetidine, amiodarone, doxepin, ticlopidine, and haloperidol, where the medication is being taken concurrently with said tamoxifen, where the presence of the HOXB13:IL17BR expression ratio greater than -1.339 or taking the medication indicates that the patient is likely to experience cancer relapse. The breast cancer patient can be a non-negative breast cancer patient. The presence of the HOXB13:IL17BR expression ratio greater than -1.339 and taking the medication can indicate that the patient is likely to experience cancer relapse. The absence of the HOXB13:IL17BR expression ratio greater than - 1.339 and not taking the medication can indicate that the patient is likely to experience a relapse-free survival or a disease-free survival. Determining whether or not the breast cancer patient taking tamoxifen contains cancer tissue with the HOXB13:IL17BR expression ratio greater than -1.339 can comprise using the HOXB13:IL-17BR calculation method.

Unless otherwise defined, all technical and scientific terms used herein have the same meaning as commonly understood by one of ordinary skill in the art to which this invention pertains.

In case of conflict, the present specification, including definitions, will control.

The details of one or more embodiments of the invention are set forth in the accompanying drawings and the description below.

### DESCRIPTION OF THE DRAWINGS

Figure 1 is a graph plotting Kaplan-Meier Estimates of Relapse-Free Survival (RFS) in the entire cohort by HOXB13:IL-17BR (> vs. < -1.849).
Figure 2 is a graph plotting Kaplan-Meier Estimates of Disease-Free Survival (DFS) in the entire cohort by HOXB13:IL-17BR (> vs. < -1.849).
Figure 3 is a graph plotting Kaplan-Meier Estimates of Overall Survival (OS) in the entire cohort by HOXB13:IL-17BR (> vs. < -1.849).
Figure 4 provides Hazard Ratios for Relapse-Free Survival (RFS), Disease-Free Survival (DFS), and Overall Survival (OS) by univariate Cox proportional hazard modeling, univariate Faraggi-Simon cross validation, multivariate Cox proportional hazards modeling, and Faraggi-Simon multivariate cross validation in all patients using HOXB13:IL-17BR (> vs. < - 1.849).
Figure 5 is a graph plotting Kaplan-Meier Estimates of Relapse-Free Survival (RFS) in the node negative cohort by HOXB 13:IL-17BR (> vs. < - 1.339).
Figure 6 is a graph plotting Kaplan-Meier Estimates of Disease-Free Survival (DFS) in the node negative cohort by HOXB13:IL-17BR (> vs. < - 1.339).
Figure 7 is a graph plotting Kaplan-Meier Estimates of Overall Survival (OS) in the node negative cohort by HOXB13:IL-17BR (> vs. < -1.339).
Figure 8 provides Hazard Ratios for Relapse-Free Survival (RFS), Disease-Free Survival (DFS), and Overall Survival (OS) by univariate Cox proportional hazard modeling, univariate Faraggi-Simon cross validation, multivariate Cox proportional hazards modeling, and Faraggi-Simon multivariate cross validation in node negative patients using HOXB 13:IL-17BR (> vs. < -1.339).

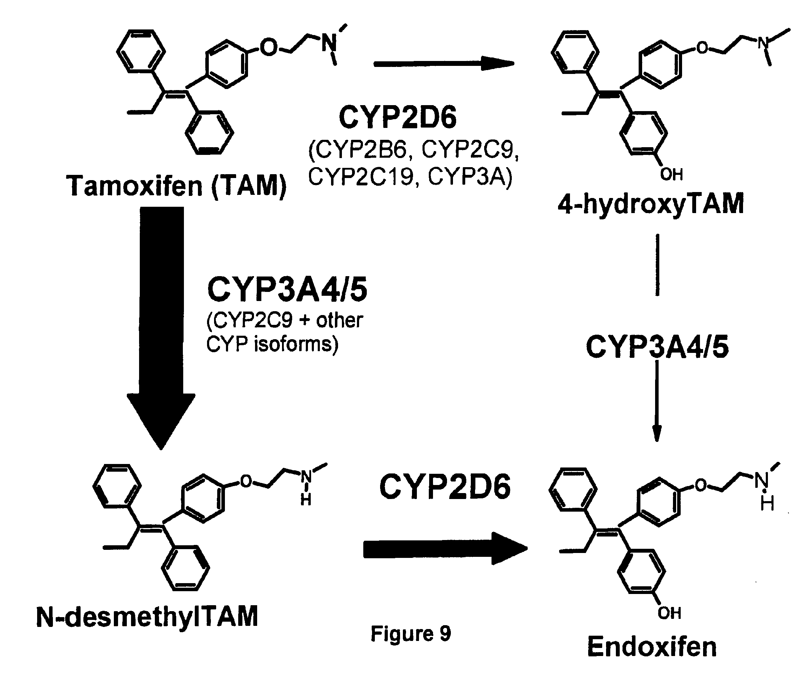

Figure 9 is a schematic diagram oftamoxifen transformation pathways listing the primary CYP enzymes involved. The relative contribution of each pathway to the overall oxidation of tamoxifen is shown by the thickness of the arrow, and the principal P450 isoforms responsible are highlighted in larger fonts.
Figure 10 is a graph plotting Kaplan-Meier Estimates of relapse-free survival for patients with the CYP2D6*4 genotype.
Figure 11 is a graph plotting Kaplan-Meier Estimates of disease-free survival for patients with the CYP2D6*4 genotype.
Figure 12 is a graph plotting Kaplan-Meier Estimates of overall survival for patients with the CYP2D6*4 genotype.
Figure 13 is a graph plotting Kaplan-Meier Estimates of relapse-free time (a), relapse-free survival (b), disease-free survival (c), and overall survival (d) based on metabolizer status (extensive, intermediate, or poor).
Figure 14 is a graph plotting smoothed hazard rates for relapse-free survival of patients with known metabolizer status (intermediate/poor versus extensive).

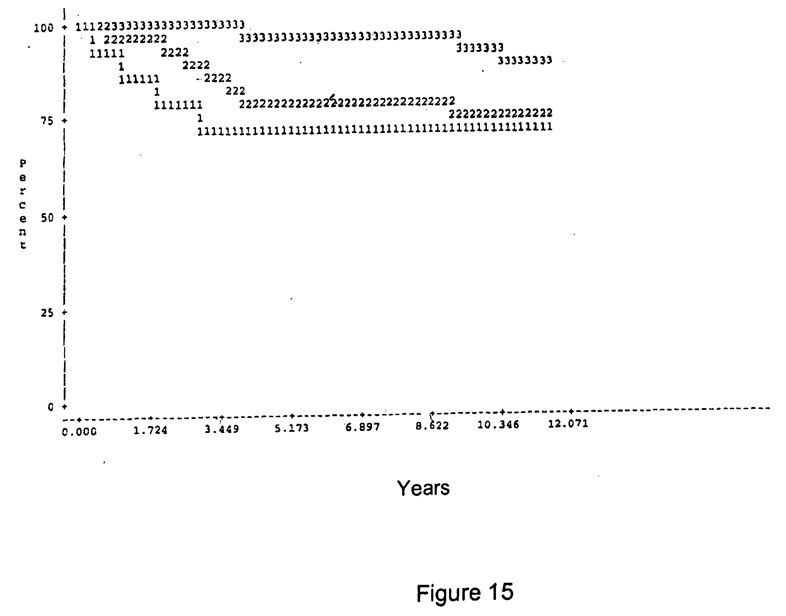

Figure 15 is a graph plotting Kaplan-Meier Estimates of time to breast recurrence in a node negative cohort by the Goetz index. The number 1 represents high risk patients, the number 2 represents intermediate risk patients, and the number 3 represents low risk patients.

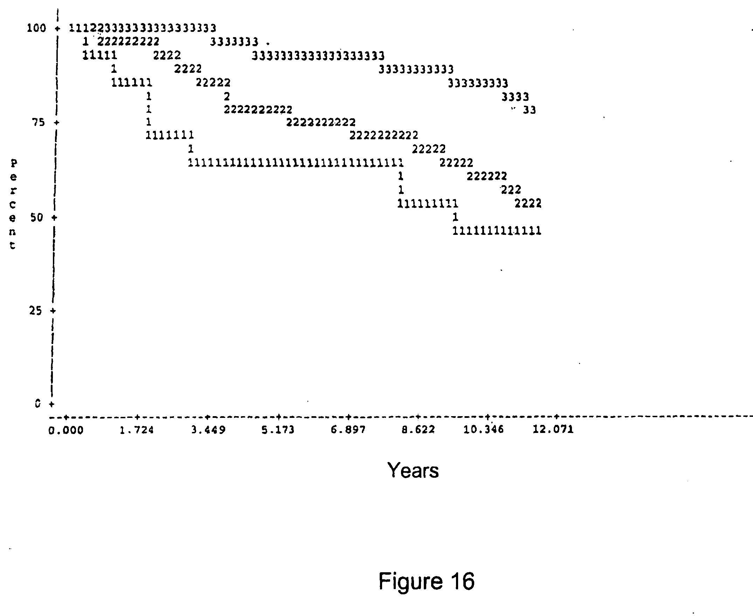

Figure 16 is a graph plotting Kaplan-Meier Estimates of relapse-free survival (RFS) in a node negative cohort by the Goetz index. The number I represents high risk patients, the number 2 represents intermediate risk patients, and the number 3 represents low risk patients.

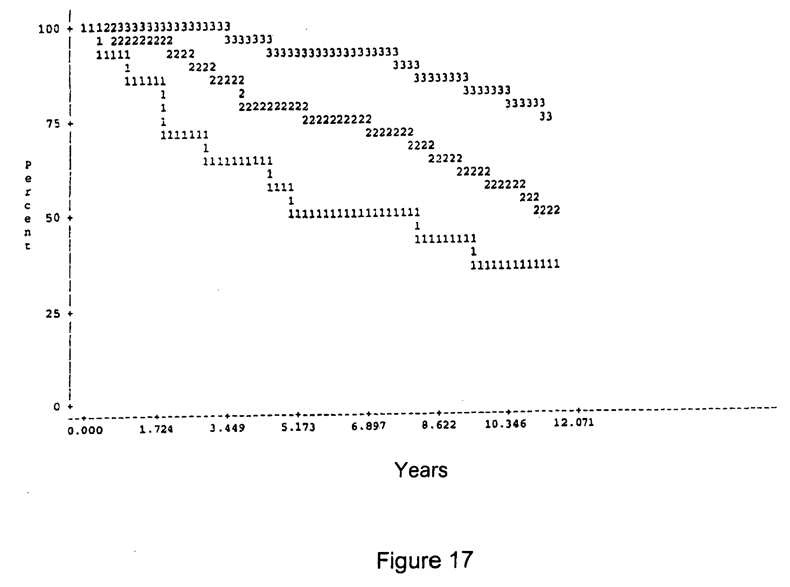

Figure 17 is a graph plotting Kaplan-Meier Estimates of disease-free survival (DFS) in a node negative cohort by the Goetz index. The number 1 represents high risk patients, the number 2 represents intermediate risk patients, and the number 3 represents low risk patients.

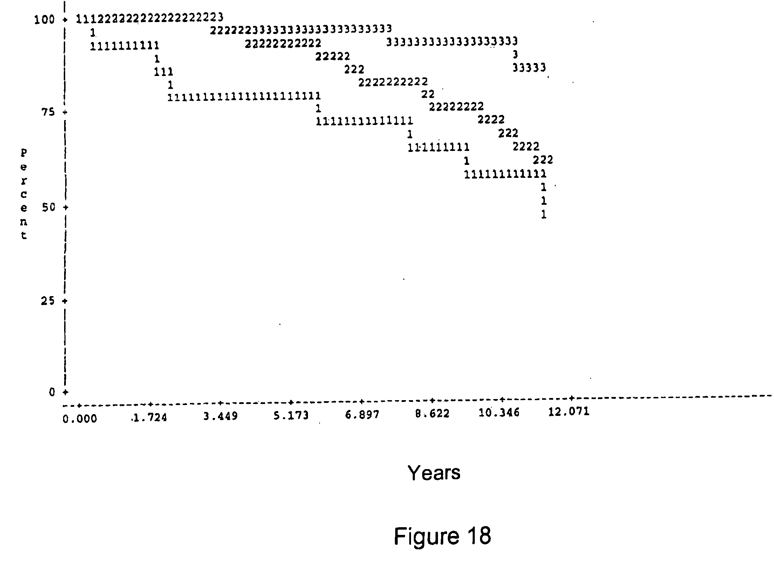

Figure 18 is a graph plotting Kaplan-Meier Estimates of overall survival (OS) in a node negative cohort by the Goetz index. The number 1 represents high risk patients, the number 2 represents intermediate risk patients, and the number 3 represents low risk patients.

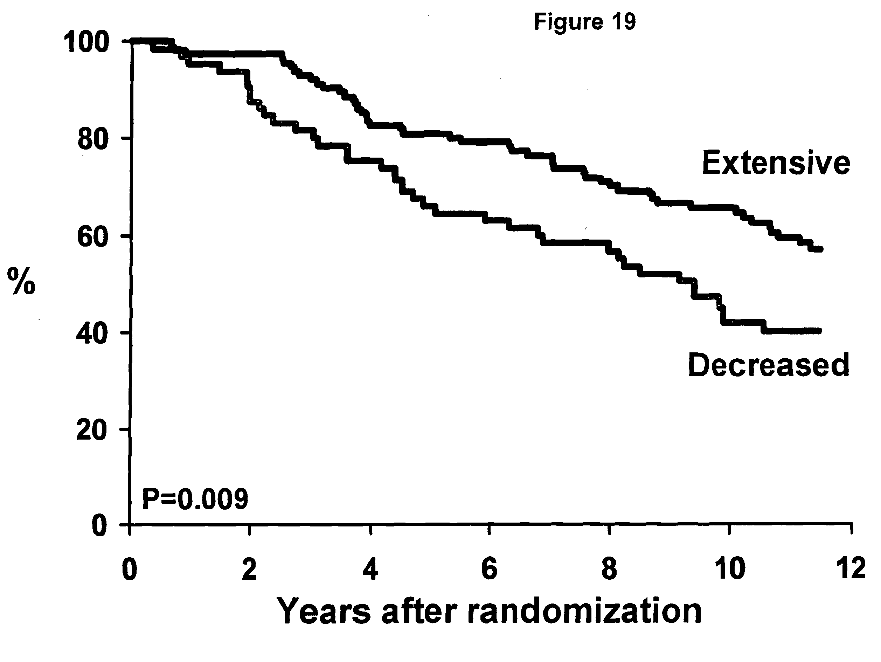

Figure 19 is a graph plotting Kaplan-Meier Estimates of disease-free survival based on CYP2D6 metabolism (extensive versus decreased) in patients with known CYP2D6 metabolism (n = 180).

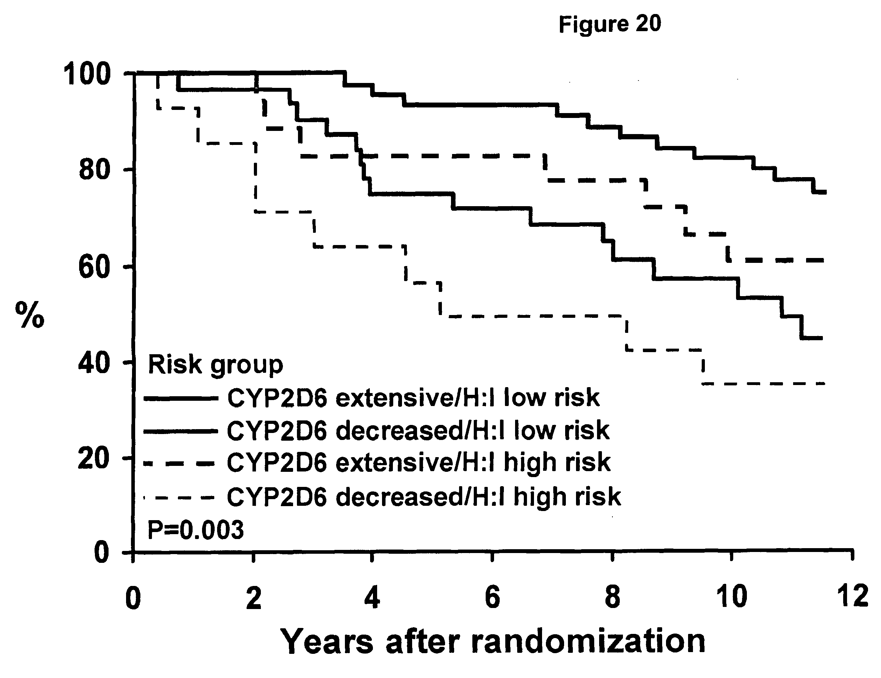

Figure 20 is a graph plotting Kaplan-Meier Estimates of disease-free survival in lymph node negative patients (n = 130) according to HOXB13/IL-17BR (> vs. < -1.339).

### DETAILED DESCRIPTION

This document provides methods and materials related to assessing the likely outcome for mammals (e.g., humans) with cancer (e.g., breast cancer). For example, this document provides methods and materials that involve assessing a breast cancer patient's (1) ratio of HOXB 13 polypeptide expression to IL-17BR polypeptide expression, (2) CYP2D6 genotype, (3) medication history, or (4) a combination thereof, to determine the likelihood of a breast cancer patient to experience breast cancer relapse or death. A breast cancer patient can be a breast cancer patient treated with tamoxifen or can be a breast cancer patient not receiving a cancer treatment. In some cases, a breast cancer patient can have node positive or node negative breast cancer.

Any method can be used to assess a patient's ratio of HOXB13 polypeptide expression to IL-17BR polypeptide expression. For example, PCR techniques such as reverse transcriptase-PCR or real-time PCR can be used to determine a patient's ratio of HOXB13 polypeptide expression to IL-17BR polypeptide expression. In some cases, immunoassays such as ELISAs or protein chip techniques can be used to determine a patient's ratio of HOXB13 polypeptide expression to IL-17BR polypeptide expression.

As used herein, the term "HOXB13:IL-17BR expression ratio greater than -1.339" refers to a ratio of HOXB 13 polypeptide expression to IL-17BR polypeptide expression that is greater than -1.339 when determined by the HOXB13:IL-17BR calculation method, that involves performing reverse transcription PCR amplification of HOXB13 RNA and IL-17BR RNA from linearly amplified RNA extracted from breast cancer cells isolated from paraffin-embedded tissue, and calculating of the difference of log₂-transformed quantities of HOXB13 RNA and IL-17BR RNA determined using standard curves and without being z-transformed or normalized to expression of a housekeeping gene.

As used herein, the term "HOXB13:IL-17BR expression ratio less than - 1.339" refers to a ratio of HOXB13 polypeptide expression to IL-17BR polypeptide expression that is less than -1.339 when determined by the HOXB13:IL-17BR calculation method.

Any appropriate method can be used to determine whether a mammal contains cancer tissue having a HOXB13:IL-17BR expression ratio greater than -1.339 or a HOXB13:IL-17BR expression ratio less than -1.339. For example, it can be determined whether a mammal contains cancer tissue having a HOXB13:IL-17BR expression ratio greater than -1.339 or a HOXB13:IL-17BR expression ratio less than -1.339 by using the HOXB13:IL-17BR calculation method.

In some cases, a method that does not involve the HOXB 13:IL-17BR calculation method can be used to determine whether a mammal contains cancer tissue having a HOXB13:IL-17BR expression ratio greater than -1.339 or a HOXB13:IL-17BR expression ratio less than -1.339. For example, a polypeptide-based method can be used to determine whether a mammal contains cancer tissue having a HOXB13:IL-17BR expression ratio greater than -1.339 or a HOXB13:IL-17BR expression ratio less than -1.339. In such a case, a mass spectrometry or an immunological assay (e.g., ELISA) can be used to analyze HOXB13 polypeptide and IL-17BR polypeptide levels in cancer tissue from a mammal. The level of HOXB13 polypeptide can be divided by the level of IL-17BR polypeptide. The result can be assessed to determine if it corresponds to a HOXB13:IL-17BR expression ratio greater than -1.339 or a HOXB13:IL-17BR expression ratio less than -1.339. Such an assessment can include comparing results obtained using the polypeptide-based method and those obtained using the HOXB13:IL-17BR calculation method for the same sample. For example, a sample known to have a HOXB13:IL-17BR expression ratio of -1.339 as determined by the HOXB13:IL-17BR calculation method can be evaluated using a polypeptide-based method to establish a comparable cut-off for that polypeptide-based method.

Another example of a method that does not involve the HOXB13:IL-17BR calculation method and that can be used to determine whether a mammal contains cancer tissue having a HOXB 13:IL-17BR expression ratio greater than -1.339 or a HOXB13:IL-17BR expression ratio less than -1.339 can be a nucleic acid-based method that involves using breast tissue from a biopsy. Such a nucleic acid-based method can include performing reverse transcription PCR amplification of HOXB13 RNA and IL-17BR RNA from total RNA extracted from breast tissue obtained by a breast biopsy procedure from a mammal. The quantity of HOXB 13 RNA can be divided by the quantity of IL-17BR RNA. The result can be assessed to determine if it corresponds to a HOXB13:IL-17BR expression ratio greater than -1.339 or a HOXB13:IL-17BR expression ratio less than -1.339. Such an assessment can include comparing results obtained using the nucleic acid-based method and those obtained using the HOXB13:IL-17BR calculation method for the same sample. For example, a sample known to have a HOXB13:IL-17BR expression ratio of -1.339 as determined by the HOXB 13:IL-17BR calculation method can be evaluated using a nucleic acid-based method to establish a comparable cut-off for that nucleic acid-based method.

Expression of any HOXB13 polypeptide and any IL-17BR polypeptide, or any HOXB13 RNA and any IL-17BR RNA, can be measured in a sample from a mammal to determine a ratio of HOXB13 polypeptide expression to IL-17BR polypeptide expression. Examples of HOXB13 polypeptides include human HOB13 polypeptides (e.g., a human HOXB13 polypeptide set forth in GenBank^{®} GI number 84043952). Examples of HOXB13 RNA molecules include human HOXB13 RNA molecules (e.g., a human HOXB13 RNA molecule set forth in GenBank^{®} GI number 84043952). Examples of IL-17BR polypeptides include human IL-17BR polypeptides (e.g., a human IL-17BR polypeptide set forth in GenBank^{®} GI number 112382255). Examples of IL-17BR RNA molecules include human IL-17BR RNA molecules (e.g., a human IL-17BR RNA molecule set forth in GenBank^{®} GI number 112382255).

Any method can be used to determine whether or not a mammal has a CYP2D6 *4/WT or a CYP2D6 *4/*4 genotype. For example, standard PCR and sequencing techniques can be used to determine the presence or absence of a CYP2D6 *4/*4 genotype.

A patient's medical history can be evaluated to determine whether or not the patient is or was taking a CYP2D6 inhibitor while concurrently taking tamoxifen. Examples of CYP2D6 inhibitors include, without limitation, fluoxetine, paroxetine sertraline, cimetidine, amiodarone, doxepin, ticlopidine, and haloperidol. In some cases, a patient can be questioned to determine whether or not the patient is or was taking a CYP2D6 inhibitor while concurrently taking tamoxifen.

As described herein, a patient can be assigned to a risk group based on the patient's (1) ratio of HOXB13 polypeptide expression to IL-17BR polypeptide expression, (2) CYP2D6 genotype, and (3) history of taking a CYP2D6 inhibitor while concurrently taking tamoxifen. In some cases, a patient can be assigned to a low, intermediate, or high risk group based on the Goetz index. In some cases, a patient can be assigned to one of the following four groups: (1) extensive CYP2D6 metabolism, low HOXB13:IL17BR expression ratio, (2) decreased CYP2D6 metabolism, low HOXB13:IL17BR expression ratio, (3) extensive CYP2D6 metabolism, high HOXB13:IL17BR expression ratio, and (4) decreased CYP2D6 metabolism, high HOXB13:IL17BR expression ratio.

This document also provides methods and materials that involve assessing the likelihood that a breast cancer patient being treated with tamoxifen will experience side effects such as hot flashes. As described herein, patients (e.g., women) with a CYP2D6 *4/*4 genotype tend to have a lower incidence of hot flashes when treated with tamoxifen than women lacking a CYP2D6 *4/*4 genotype.

Examples not pertaining to the invention serve for illustrative purposes only.

### Example 1 - Assessing breast cancer outcomes using HOXB13:IL-17BR expression ratios

### Materials and Methods

***Patients.*** The North Central Cancer Treatment Group conducted a randomized phase III clinical trial in postmenopausal women with resected ER positive breast cancer to assess the value of adding one year of fluoxymesterone to 5 years of tamoxifen adjuvant therapy (NCCTG 89-30-52; Ingle et al., Proc. Am. Soc. Clin. Oncol., 19:86a (2000)). Postmenopausal women with node-negative disease were required to have stage T₁c or T₂N₀M₀ cancer and could be any age, whereas women with node-positive disease were required to be at least 65 years of age with a tumor stage T₁ (any N) or T₂N₁M₀. A woman was classified as postmenopausal if one of the following held: (1) her last menstrual cycle was > 12 months prior to diagnosis, (2) her last menstrual cycle was 4-12 months prior to diagnosis and her follicle-stimulating hormone (FSH) level was in the postmenopausal range, (3) she had a bilateral oophorectomy at least 2 months prior to diagnosis, or (4) she had a hysterectomy without oophorectomy and was either > 60 years old or her FSH level was in the postmenopausal range. Patients were surgically treated with either a modified radical mastectomy or breast conservative therapy including lumpectomy, axillary nodal dissection, and radiation therapy. The axillary dissection must have involved at least levels I and II and the examination of at least 6 axillary nodes. Patients who underwent lumpectomy must have had a primary tumor no larger than 5 cm, and the surgical margins must have been microscopically free of tumor. Postlumpectomy radiation therapy consisted of a total cumulative breast dose of 5040 cGy in 28 fractions, and those with axillary nodal involvement also received radiation to the axilla and supraclavicular regions. Patients were classified as ER positive if resected breast tissue contained ≥ 10 fmol of ER polypeptide / mg cytosol protein or was positive for ER polypeptide by an immunohistochemical assay. All patients were randomized within 6 weeks of definitive surgery.

A total of 541 patients were randomized to either oral tamoxifen, 20 mg daily for 5 years (256 eligible) or tamoxifen, 20 mg daily for 5 years plus oral fluoxymesterone, 10 mg twice daily for one year (258 eligible). Patients were stratified based on axillary lymph node status (0, 1-3, 4-9 vs. 10 or more), age (< 65 years vs. > 65), primary tumor size (< 3 cm vs. >3 cm), ER status (10-49 fmol vs. 50 fmol or greater vs. positive by immunohistochemical assay), and extent of surgery (mastectomy vs. breast conservation therapy).

Clinical evaluations including history, physical examination, blood and chemistry groups, chest x-ray, and toxicity assessments were performed every 4 months for the first year, every 6 months during years 2-5, and then yearly. Mammograms and pelvic examinations were performed annually. Toxicities were graded using the NCI Common Toxicity Criteria version 1.0 and the NCCTG supplement.

Within 30 days of registration, a paraffin embedded tumor block was submitted for future research purposes.

***Tissue Preparation and RNA amplification.*** Utilizing the available 227 paraffin-embedded tissue blocks for the women enrolled in the tamoxifen only arm, one hematoxylin and eosin slide and two 7 µm sections were cut from each paraffin embedded block, mounted on PEN membrane glass slides (Microdissect Gmbh, Germany), and stained using the Histogene^{™} Staining kit (Arcturus Bioscience, Molecular Devices, Sunnyvale, CA). Two breast cancer pathologists independently verified the presence of invasive breast cancer in 211 of the 227 paraffin blocks and provided Nottingham tumor grade for both invasive ductal and lobular carcinomas. In the case of assigning a score for tubule formation, the pathologist automatically assigned lobular carcinomas a score of 3 (<10% tubule formation). Tumor regions were isolated using laser-cutting and subsequently captured by using the Arcturus Veritas^{™} Laser Microdissection System (Arcturus Bioscience, CA) and immediately placed in proteinase K buffer (Paradise^{™} Reagent System, Arcturus Bioscience, CA). The proteinase K lysates were incubated for 16 hours at 50°C, and total RNA was purified and subjected to two rounds of linear amplification as described by the manufacturer (Paradise^{™} Reagent System) to obtain amplified RNA (aRNA).

***Real-time PCR.*** TaqMan^{®} primers and probes were designed using Primer Express (Applied Biosystems, Foster City, CA). The primer sequences for HOXB13 were 5'-GCCATGATCGTTAGCCTCATATT-3' (forward primer; SEQ ID NO: 1) and 5'-CAATTCATGAAAGCGGTTTCTAAAG-3' (reverse primer; SEQ ID NO:2) with a minor groove binder (MGB) probe sequence VIC-TCTATCTAGAGCTCTGTAGAGC-MGB (SEQ ID NO:3); the primer sequences for IL-17BR were 5-GGCTTCCTA-TCCCACCAATT-3' (SEQ ID NO:4) and 5'-AGGCTGTTTGTAGGCTGCA-3' (SEQ ID NO:5) with an MGB probe sequence VIC-CAGGGAAAAAACGTGTGATG-MGB (SEQ ID NO:6).

An aliquot (200-500 ng) of the amplified RNA (aRNA) from each tumor sample was converted into cDNA via reverse transcription using the Paradise Reagent System. TaqMan^{®} assays were performed in duplicate using 1/30^{th} of the reverse transcribed material in a 20 µL reaction volume in a 384-well plate using the ABI 7900HT instrument (Applied Biosystems, CA). The samples were heated to 50°C for 2 minutes, 95°C for 10 minutes, followed by 45 cycles of 95°C for 15 seconds and 60°C for 1 minute. For each gene, a standard curve was constructed with cDNA dilutions derived from amplified human universal total RNA (Stratagene, La Jolla, CA) to obtain relative expression levels (i.e., quantities) of HOXB13 and IL-17BR. The HOXB 13:IL-17BR ratio was obtained as the difference of log₂-transformed quantities of HOXB 13 and IL-17BR. No control genes were measured, as the direct ratio calculation does not require a normalization factor. To control for plate to plate variation of the PCR reaction, standard curves were run on each plate.

***Study Design and End Points.*** One objective of this study was to examine the relationship between the HOXB13:IL-17BR expression ratio and clinical outcomes of relapse-free survival, disease-free survival, and overall survival. Relapse-free survival (RFS) was defined as the time from randomization to documentation of the first of the following events: any recurrence (local, regional or distant) of breast cancer, a contralateral breast cancer or death. When estimating the distribution of RFS, patients who developed a non-breast second primary cancer (other than squamous or basal cell carcinoma of the skin, carcinoma *in situ* of the cervix, or lobular carcinoma *in situ* of the breast) prior to the diagnosis of a breast event were censored on the day their second primary was diagnosed. Patients who were alive without a breast recurrence, contralateral breast cancer or a second non-breast primary cancer were censored at the date of their last disease evaluation. Disease-free survival (DFS) was defined as the time from randomization to documentation of the first of the following events: any recurrence (local, regional or distant) of breast cancer, a contralateral breast cancer, a second primary cancer, or death due to any cause. Patients who were alive without any of these events were censored at the date of their last disease evaluation. Overall survival (OS) was estimated as the time from registration to death due to any cause.

To assess whether clinical outcome differed with respect to the HOXB13:IL-17BR expression ratio, a minimum p-value approach was used to identify a cut-point for the HOXB13:IL-17BR expression ratio that best discriminates between those patients with a poor clinical outcome and those patients with a better clinical outcome. For each clinical outcome, this 'optimal' cut-point was sought from among the observed values of the expression ratio above the 10^{th} percentile and below the 90^{th} percentile of the expression ratio distribution. To account for multiple testing, a correction to the p-value associated with the 'optimal' cut-point was employed as proposed by Lausen and Schumacher (Biometrics, 1992:73-85 (1992)) and modified by Altman et al. (J. Natl. Cancer Inst., 86(11):829-35 (1994)). The resulting p-value, denoted as p_{cor}, and uncorrected log-rank p-value are reported. Because inclusion of a biomarker dichotomized at its 'optimal' cut-point in a Cox regression analysis may inflate the effect (Lausen and Schumacher, Biometrics, 1992:73-85 (1992); Altman et al., J. Natl. Cancer Inst., 86(11):829-35 (1994); Hilsenbeck et al., Breast Cancer Res. Treat., 22(3):197-206 (1992); Simon and Altman, Br. J. Cancer, 69(6):979-85 (1994); and Faraggi and Simon, Stat. Med., 15(20):2203-13 (1996)), the cross-validation approach of Faraggi and Simon was used to obtain a point and interval estimate of the hazard of each clinical outcome for those with a high HOXB13:IL-17BR expression ratio relative to those with a low HOXB13:IL-17BR expression ratio.

Log rank tests and univariate Cox proportional hazard models were used to assess whether the distributions of RFS, DFS, or OS differed with respect to any one of the following factors: age 65 years or greater (yes vs. no), extent of surgery (mastectomy vs. breast conserving), estrogen receptor status (10-49 fmols vs. ≥50 fmols vs. positive by immunohistochemistry), number of positive nodes (represented as three indicator variables for 1-3, 4-9, and ≥10 positive nodes), tumor size 3 cm or greater (yes vs. no), Nottingham grade (3 vs. 1 or 2), Her2 expression (3+ vs. 0, 1+, or 2+), and prior exposure to exogenous estrogens (yes vs. no). For each clinical outcome, multivariate Cox proportional hazard modeling was performed to obtain a subset of the potential prognostic factors which provided an adequate fit to the data. Residual plots were examined. The likelihood ratio test was then applied to assess whether HOXB13:IL-17BR expression ratio dichotomized at its 'optimal' cut-point made a significant contribution to the model. The cross-validation approach (Faraggi and Simon, Stat. Med., 15(20):2203-13 (1996)) was used to assess the impact of expression ratio on RFS, DFS, or OS after known prognostic factors have been accounted for. Finally, the prognostic value of the two-gene expression ratio was assessed in the node negative and node positive breast disease cohorts separately, using the same analysis approach.

### Results

***Characteristics of the Patients.*** Of the 256 eligible women enrolled to the tamoxifen only arm, 211 paraffin-embedded tumor blocks were available for RNA extraction. The relative expression levels of HOXB 13 and IL-17BR were obtained for 206 of these 211 patients. Table I presents the pre-registration characteristics for patients with and without gene expression data. The overall patient characteristics were similar, although a higher percentage of patients with HOXB 13/IL-17BR expression ratio data had a tumor size greater than 3 cm (24%) compared with the group without HOXB13/IL-17BR expression ratio data (10%).

**Table 1: Pre-registration characteristics of the patients randomized to the tamoxifen arm that did and did not have expression ratio data.**

| | Women with expression ratio data (n=206) | Women without expression ratio data (n=50) |
|---|---|---|
| **Race** | | |
| Caucasian | 92% | 91% |
| **Age** | | |
| Median (range) | 68 (42-84) | 68 (48-87) |
| **Operative procedure** | | |
| Mastectomy | 83% | 74% |
| Breast Conservation | 17% | 26% |
| **Number of Positive Nodes** | | |
| 0 | 63% | 62% |
| 1-3 | 26% | 15% |
| 4-9 | 7% | 15% |
| 10+ | 4% | 6% |
| **Tumor Size** | | |
| < 3 cm | 76% | 90% |
| ≥ 3 cm | 24% | 10% |
| **ER status** | | |
| 10-49 fmols | 20% | 20% |
| ≥ 50 fmols | 69% | 56% |
| Positive | 11% | 24% |
| **HER2** | | |
| 0 | 11% | |
| 1 | 36% | Not determined |
| 2 | 34% | |
| 3 | 18% | |
| unknown | <1% | |
| **Histology** | | |
| Ductal | 86% | |
| Lobular | 10% | Not determined |
| Other | 4% | |
| **Nottingham Tumor Grade** | | |
| Grade 1 | 26% | |
| Grade 2 | 55% | Not determined |
| Grade 3 | 18% | |
| Unknown | <1% | |

For the group of patients with gene expression data available, the first documented event was as follows: local, regional or distant breast recurrence (39 patients), contralateral breast cancer (12 patients), a second non-breast primary cancer (13 patients), both a breast recurrence and a second non-breast primary cancer (1 patient), and death without a breast recurrence or second primary cancer (37 patients). At last follow-up, 104 women were alive without evidence of a breast event or second primary, 25 were alive following a breast event or second primary cancer, 29 died with disease recurrence, 8 died having developed a second primary cancer, 29 died of other causes, and 8 died of unknown causes. The Kaplan-Meier estimates for the 5 year RFS, DFS and OS were as follows: 75.6% (95% CI: 69.1-80.9%), 74.3% (95% CI: 67.7-79.7%), and 78.2% (95% CI: 71.9-83.3%), respectively. The median length of follow-up among the 129 patients still alive was 11.0 years (range: 5.7-13.6 yrs).

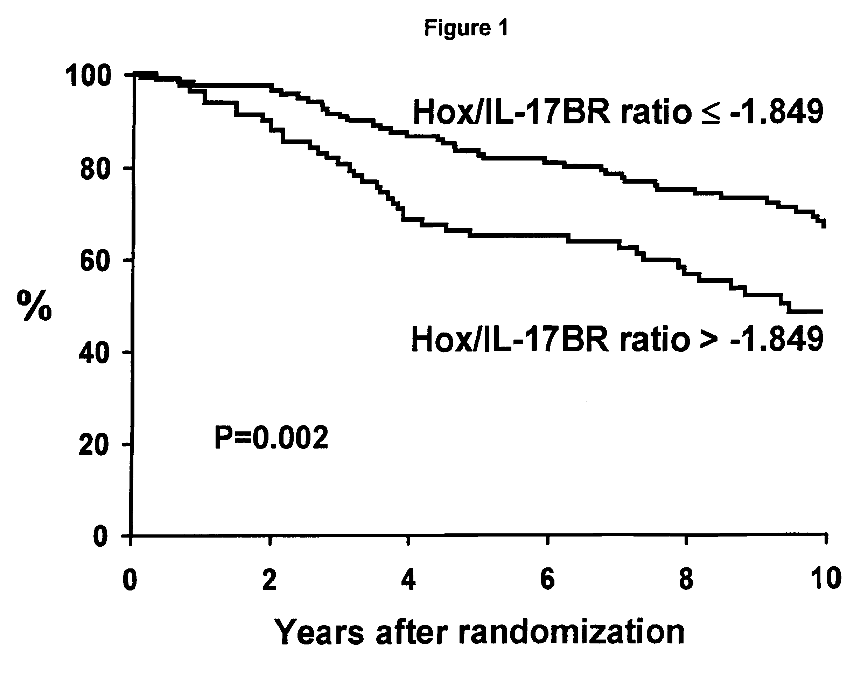

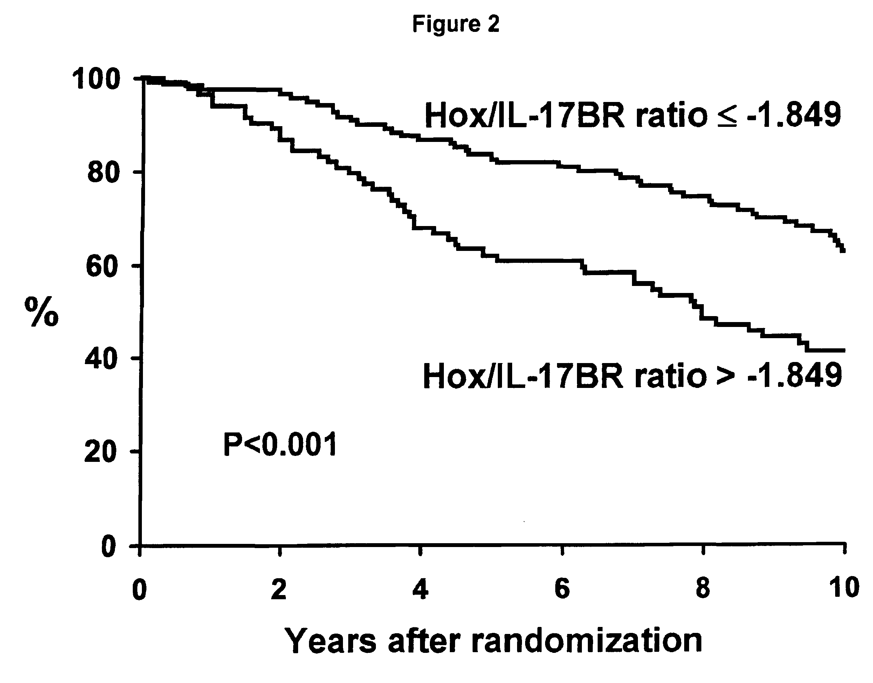

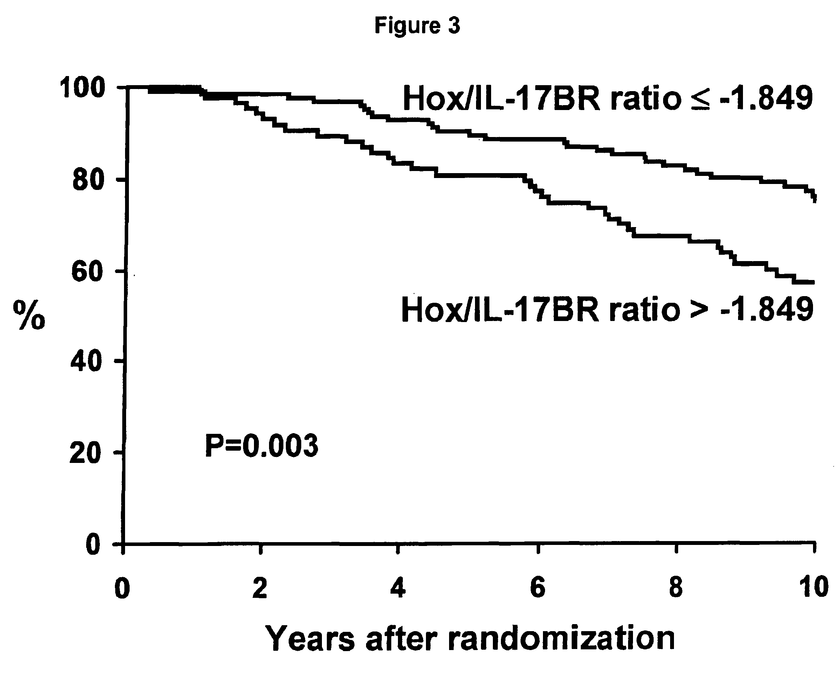

***The HOXB13:IL-17BR expression ratio cut-point.*** The cut-point for the log (HOXB13:IL-17BR expression ratio) that best discriminated clinical outcome (recurrence and survival) fell at the 58^{th} percentile of the observed HOXB13:IL-17BR expression ratio distribution (-1.849). This cut-off provided a classification which divided the women into two groups with significantly different DFS (p_{unc}<0.001), RFS (p_{unc}=0.002), and OS (p_{unc}=0.001). The Kaplan-Meier curves for RFS, DFS and OS using the cut point of -1.849 are shown in Figures 1-3. After applying the Altman method to correct for multiple testing, RFS (p_{cor}=0.044), DFS (p_{cor}<0.001), and OS (p_{cor}=0.025) still differed with respect to the HOXB13:IL-17BR expression ratio cut point of -1.849. When using the Faraggi and Simon cross-validation method in the univariate Cox model, RFS (HR_{FS}=1.62, 95% CI: 1.06-2.48; p_{FS} =0.027), DFS (HR_{FS}=1.69, 95% CI: 1.14-2.51; p_{FS} =0.009), but not OS (HR_{FS}=1.55, 95% CI: 0.98-2.45; p_{FS} =0.060) differed with respect to HOXB13:IL-17BR expression ratio (< or > - 1.849).

***Assessing the added value of HOXB13:IL-17BR expression ratio.*** For each endpoint (RFS, DFS, and OS), Cox proportional hazard modeling was performed utilizing traditional patient and tumor prognostic factors. Nodal status (positive vs. negative), tumor size (≥ 3cm vs. < 3cm), and Nottingham grade (3 vs. else) were significantly associated with each of these endpoints. When adjusting for these factors, women with a HOXB 13:IL-17BR expression ratio > - 1.849 had significantly worse RFS (HR 1.63, 95% CI: 1.05-2.53, p=0.030), DFS (HR 1.75, 95% CI: 1.16-2.63; p=0.008), and OS (HR 1.63, 95% CI: 1.63-2.60, p=0.041), independent of tumor size, nodal status and tumor grade, than women with a HOXB13:IL-17BR ratio <-1.849 (Table 2).

**Table 2: Results of Cox modeling of RFS, DFS, and OS in Entire Patient Cohort: Hazard Ratios (and corresponding 95% confidence intervals).**

| Factor | Clinical Outcome | | |
|---|---|---|---|
| | RFS | DFS | OS |
| Positive Nodes | 2.31 (1.50-3.54) | 2.22 (1.49-3.31) | 2.41 (1.54-3.79) |
| Tumor size | 1.93 (1.23-3.03) | 1.98 (1.31-3.00) | 2.01 (1.26-3.21) |
| Tumor Grade | 1.88 (1.13-3.14) | 1.69 (1.04-2.75) | 1.88 (1.11-3.18) |
| HOXB13:IL-17BR expression ratio | 1.63 (1.05-2.53) | 1.75 (1.16-2.63) | 1.63 (1.02-2.60) |

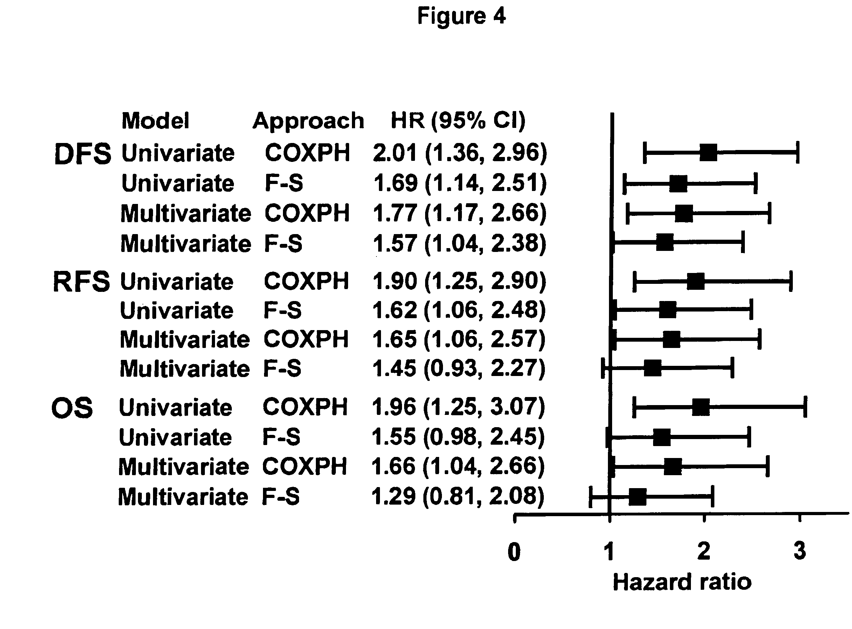

The Faraggi and Simon cross-validation method in the multivariate analysis was applied. It was found that although women with a HOXB13:IL-17BR expression ratio > -1.849 had significantly worse DFS (HR 1.57, 95% CI: 1.04-2.38; p=0.03) compared with a HOXB13:IL-17BR expression ratio < - 1.849, there were no significant differences with respect to RFS (HR=1.45, 95% CI: 0.93-2.27; p=0.100) or survival (HR=1.29, 95% CI: 0.81-2.08; p=0.284) when tumor size, nodal status, and tumor grade were accounted for in this model. Figure 4 shows a forest plot for each of the statistical analyses demonstrating the hazard ratio and corresponding 95% confidence intervals for RFS, DFS, and OS using the HOXB13:IL-17 cut point of -1.849.

***The HOXB13:IL17BR expression ratio and nodal status.*** The distribution of the HOXB13:IL17BR ratio was assessed by nodal status. The median ratio was found to be similar when comparing node positive (median - 3.81; range -10.15 to 9.37) with node negative patients (median -2.73; range - 11.04 to 7.79), indicating that the assay performed well for both populations. Because the HOXB13 gene was previously determined to affect cell migration and invasion (Ma et al., Cancer Cell., 5(6):607-16 (2004)), the cut-point that best discriminated clinical outcome may differ as a function of nodal status. Therefore, the optimal cut points in the node negative (n=130) and node positive (n=96) cohorts were separately determined.

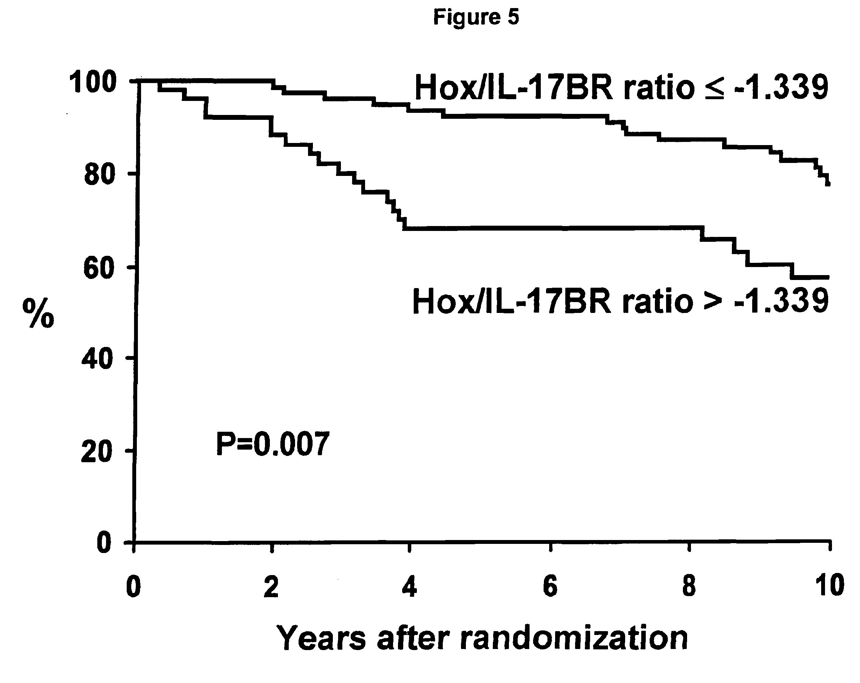

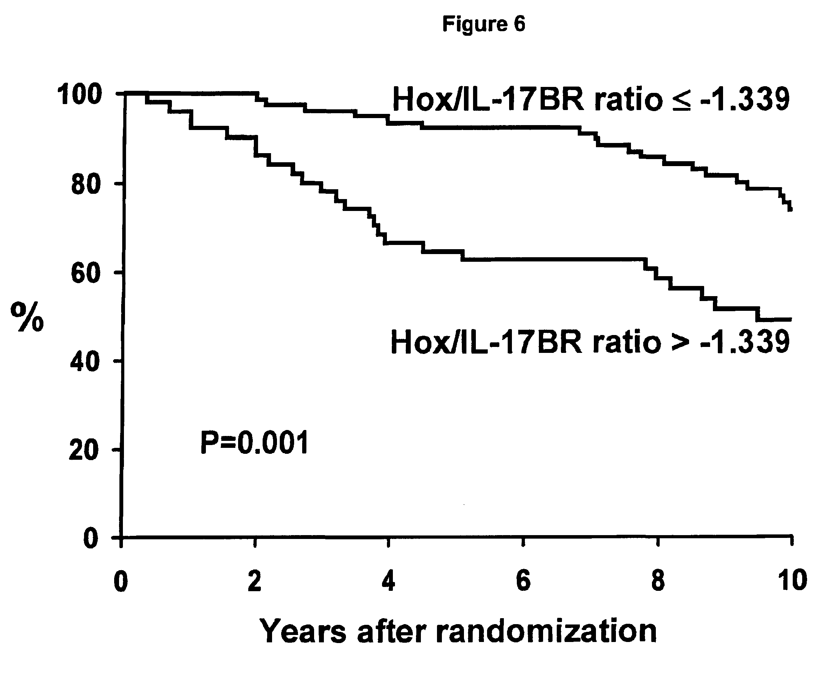

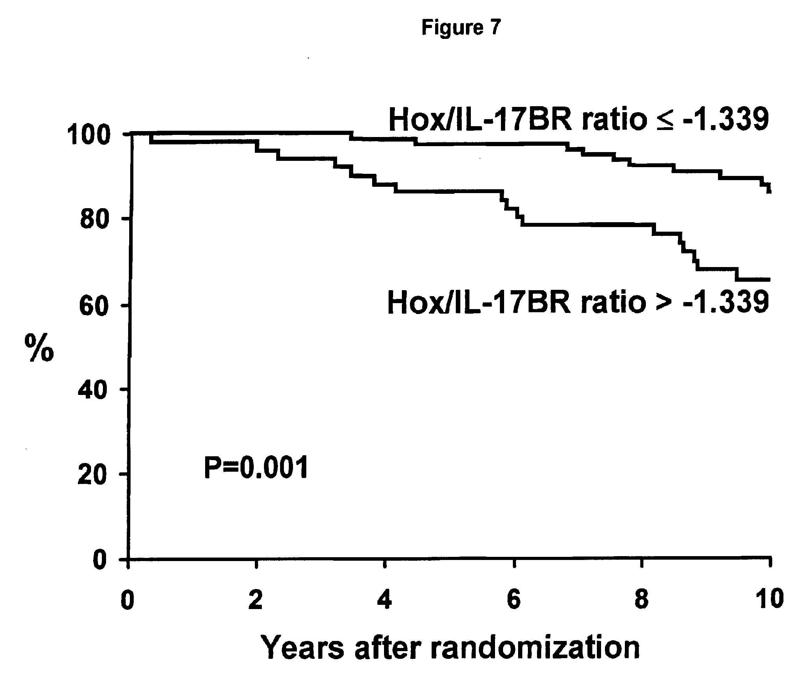

*Node Negative* Disease Among the 130 patients diagnosed with node negative disease, the 'optimal' cut-point fell at the 59^{th} percentile of the expression ratio distribution (-1.339). This cut-off provided a classification which divided the women into two groups with significantly different DFS (p_{unc}=0.001), RFS (p_{unc}=0.007), and OS (p_{unc}<0.001). The Kaplan-Meier curves are shown in Figures 5-7, and demonstrate that node negative patients with a HOXB13:IL17BR ratio of > -1.339 have significantly worse RFS, DFS, and OS compared with patients with a ratio of less than -1.339. After applying the Altman method to correct for multiple testing, DFS (p_{cor}=0.025), OS (p_{cor}=0.003), but not RFS (p=0.282), were found to differ with respect to HOXB13:IL17BR expression ratio cut point of -1.339. However, when the Faraggi and Simon cross-validation method was applied using a univariate Cox model, all three endpoints including RFS (HR_{FS}=1.99, 95% CI: 1.09-3.63; p_{FS} =0.025), DFS (HR_{FS}=2.12, 95% CI: 1.22-3.68; p_{FS} =0.008), and OS (HR_{FS}=2.35, 95% CI: 1.21-4.58; p_{FS} =0.012) differed with respect to HOXB13:IL17BR expression.

For each endpoint, Cox proportional hazard modeling was performed utilizing traditional patient and tumor prognostic factors in the node negative cohort. Only tumor size (≥ 3cm vs. < 3cm) was significantly associated with each of these endpoints. The HOXB13:IL17BR expression ratio of -1.339 was used, and the likelihood ratio test was applied to assess whether the expression ratio was significantly associated with DFS, RFS, or OS. Patients with a HOXB 13:IL 17BR expression ratio > -1.339 had significantly worse RFS (HR 1.98, 95% CI: 1.07-3.68; p=0.031), DFS (HR 2.03, 95% CI: 1.15-3.59; p=0.015), and OS (HR 2.4, 95% CI: 1.19-4.84; p=0.014) independent of tumor size, compared with patients with a HOXB13:IL17BR expression ratio < -1.339 (Table 3).

**Table 3: Results of Cox modeling of RFS, DFS, and OS in Node-Negative Patient Cohort: Hazard Ratios (and corresponding 95% confidence intervals).**

| Factor | Clinical Outcome | | |
|---|---|---|---|
| | RFS | DFS | OS |
| Tumor size | 1.83 (0.92-3.65) | 2.38 (1.30-4.36) | 2.48 (1.22-5.06) |
| HOXB13 :IL-17BR expression ratio | 1.98 (1.07-3.68) | 2.03 (1.15-3.59) | 2.40 (1.19-4.84) |

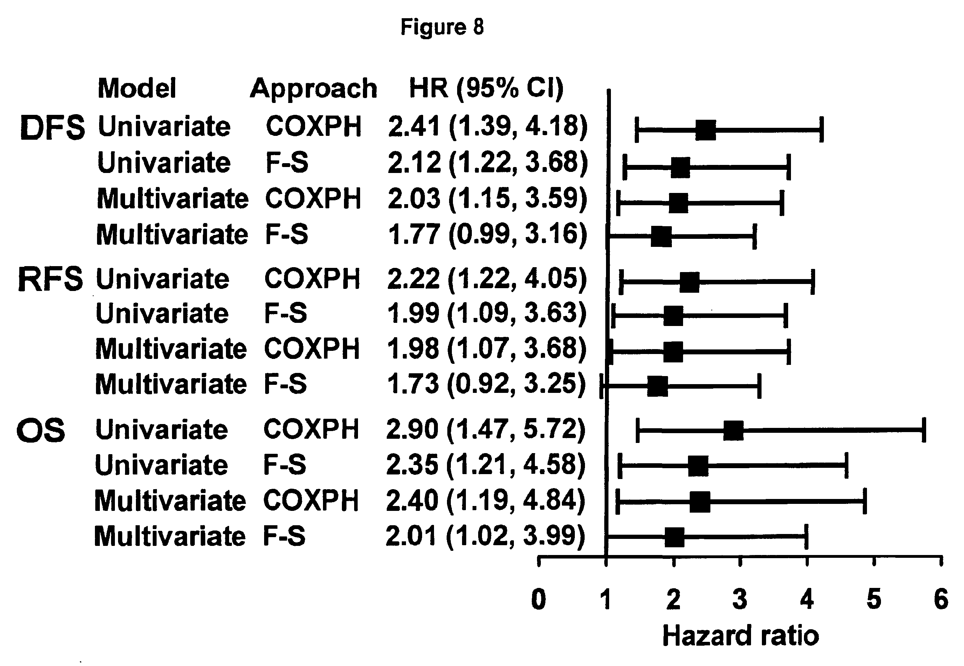

Finally, the Faraggi and Simon cross-validation method in the multivariate analysis was applied. Women with a HOXB13:IL17BR expression ratio > -1.339 tended to have worse RFS (HR=1.72, 95%CI: 0.92-3.25; p=0.088), DFS (HR=1.77, 95%CI: 0.99-3.16; p=0.054), and statistically significantly worse OS (HR=2.01, 95%CI 1.02-3.99; p=0.045), compared with patients with an expression ratio < -1.339. Figure 8 shows a forest plot for each of the statistical analyses demonstrating the HR and corresponding 95% CI for RFS, DFS, and OS using the HOXB13:IL17BR cut point of -1.339.

*Node Positive Disease.* Among the 96 patients diagnosed with node positive disease, the 'optimal' cut-point was at the far right of the expression ratio distribution (namely, the 90^{th} percentile (4.4). Both the Altman approach and Faraggi and Simon procedure led to the conclusion that there was no evidence to suggest that RFS (p_{cor} = 0.217, p_{FS} = 0.120), DFS (p_{cor} = 0.148, p_{FS} = 0.069), or OS (p_{cor} = 0.148, p_{FS} = 0.324) differs with respect to the HOXB13:IL17BR expression ratio.

The results provided herein by studying a cohort of postmenopausal women with tamoxifen treated breast cancer demonstrate that the HOXB13 to IL-17BR gene expression ratio is associated with breast cancer recurrence and survival, independent of standard clinical and pathological prognostic markers. Furthermore, these results demonstrate that this marker can be used in the node negative breast cancer patient population. Using statistical cross validation, it was demonstrated that in the node negative cohort, a high HOXB13 to IL-17BR ratio was associated with worse survival in the univariate (p<0.0001), the univariate Cox cross validation model (p=0.012), multivariate (HR 2.4, 95% CI 1.19-4.84, p=.014), and multivariate cross validation analysis (HR 2.01; 95% CI 1.02-3.99; p=0.045).

The results that a high HOXB13 to IL-17BR expression ratio is associated with a greater risk of relapse and death in node negative ER positive breast cancer but not node positive suggest that this biomarker may be a marker of early invasion and metastatic potential. This notion is further supported by the fact that the recurrences seen in patients with a high HOXB 13 to IL17-BR ratio occurred quickly, within the first four years of beginning tamoxifen, followed by a plateau until year 8, at which time further relapses were seen in both arms (Figure 5). In women with negative lymph nodes and a HOXB13:IL-17BR ratio of less than -1.339, there were no events (recurrence or death) within the first 2 years after randomization. Therefore, the gene expression ratio may identify the biologic underpinnings for the early peak in the hazard rate for relapse, typically seen 18-24 months following initiation of hormonal therapy (Baum et al., Proc. Am. Soc. Clin. Oncol., 23(16S):31S (2005)).

The demonstration that the two gene assay is associated with poorer outcomes in node negative ER positive breast cancer but not node positive breast cancer illustrates the complexity of performing biomarker studies in patients with breast cancer. Multiple gene expression profiling studies have been published which correlate a specific profile with breast cancer outcomes; however, some of these profiles were derived from patients treated with multiple different therapies (for example either chemotherapy alone or with/without hormonal therapy) for varying stages (I-III) of pre and postmenopausal estrogen positive and negative breast cancer. In contrast, the 2-gene profile was discovered and tested in patients with ER positive breast cancer treated with tamoxifen monotherapy. This is important, because for ER positive breast cancers, it is less likely that clinicians will use gene expression profiling to exclude patients from hormonal therapy, given that hormonal therapy not only reduces the risk of distant recurrence, but also prevents the development of contralateral breast cancer (Howell et al., Lancet, 365(9453):60-2 (2005)). However, in the case of node negative ER positive breast cancer, the 2 gene biomarker may identify a high risk group of patients for which upfront aromatase inhibitors and/or chemotherapy may prevent some of the immediate recurrences seen within the first 5 years with tamoxifen monotherapy.

In summary, the results provided herein demonstrate that the HOXB13 to IL-17BR gene expression ratio is associated with relapse and survival in node negative breast cancer. Studies using untreated breast cancer patients can confirm that the 2-gene expression ratio provided herein can be used as a prognostic marker. In addition, the 2-gene expression ratio provided herein can be used to determine whether alternative hormonal therapy (e.g., aromatase inhibitors) or chemotherapy will improve the outcomes of women identified to be at high risk by means of the HOXB13-IL-17BR ratio.

### Example 2 - Assessing breast cancer outcomes using CYP2D6 genotype

### Methods and materials

*Patients.* The North Central Cancer Treatment Group (NCCTG) conducted a randomized phase III clinical trial in postmenopausal women with resected ER positive breast cancer to assess the value of adding one year of fluoxymesterone to 5 years of tamoxifen adjuvant therapy (NCCTG 89-30-52; Ingle et al., Proc. Am. Soc. Clin. Oncol., 19:86a (2000)). No women received adjuvant chemotherapy. The details of the clinical trial including the eligibility requirements were as described in Example 1.

Contraindications for entry onto protocol included pectoral fascia invasion, bilateral or previous breast cancer, cancer other than breast cancer with the exception of resected non-melanoma skin cancer or adequately treated carcinoma *in-situ* of the uterine cervix unless disease-free for at least 5 years, white blood cell count less than 3000/µL, platelet count less than 100,000/µL, total bilirubin or SGOT over 1.5 times the institutional upper limit of normal (IULN), creatinine > 2 times the IULN, warfarin therapy, and prior systemic therapy for breast cancer with the exception of tamoxifen administered within 14 days of randomization.

A total of 541 patients were randomized to either oral tamoxifen, 20 mg daily for 5 years (256 eligible) or tamoxifen, 20 mg daily for 5 years plus oral fluoxymesterone, 10 mg twice daily for one year (258 eligible). Patients were stratified based on axillary lymph node status (0, 1-3, 4-9 vs. 10 or more), age (< 65 years vs. > 65), primary tumor size (< 3 cm vs. >3 cm), ER status (10-49 fmol vs. 50 fmol or greater vs. positive by immunohistochemical assay) and local therapy (mastectomy vs. breast conservation therapy).

Clinical evaluations including history, physical examination, blood and chemistry groups, chest x-ray, and toxicity assessments were performed every 4 months for the first year, every 6 months during years 2-5, and then yearly. Mammograms and pelvic examinations were performed annually. Toxicities were graded using the NCI Common Toxicity Criteria version 1.0 and the NCCTG supplement in which hot flashes were graded as 0-none or no change, 1-mild, 2-moderate, or 3-severe.

*Sample Preparation.* Utilizing paraffin-embedded tissue blocks from the women enrolled to the tamoxifen only arm, three sections (10-µm thick) and one hematoxylin and eosin slide were prepared from each block, and a 1 cm area of high tumor cellularity was identified, microdissected, and deparaffinized. DNA was extracted using the method described elsewhere (Rae et al., Pharmacogenetics, 13:501-7 (2003)). In those living women that supplied a buccal sample, DNA was extracted using a QIAamp DNA Mini kit (Qiagen, Valencia, CA) according to the manufacturer's instructions.

*Assay methods.* Patient samples (both tumor and buccal) were genotyped for CYP2D6*4 (1846G>A), and CYP2D6*6 (1707T>del) polymorphisms as described elsewhere (Jin et al., J. Natl. Cancer Inst., 97:30-9 (2005)) using the Applied Biosystems' Taqman^{®} Allelic Discrimination Assay (Foster City, CA) according to the manufacturer's instructions. Briefly, 10 ng of DNA were added to a 5 µL reaction containing forward and reverse primers along with 2 allele specific labeled probes (one wild-type and one variant allele specific). The PCR and fluorescence measurements were performed using the ABI Prism 770 sequence detection system. Additional CYP2D6 alleles less common in the Caucasian population (*3 and *5) were not analyzed because of inadequate amounts of DNA were available for testing.

Additionally, patient samples (both tumor and buccal) were genotyped for the CYP3A5*3 polymorphism (6986 G>A) using the method described elsewhere (Hustert et al., Pharmacogenetics, 11:773-9 (2001)) with minor modifications. Briefly, a 280 base pair (bp) product was amplified using an initial 10 minute incubation at 94°C, followed by 45 cycles of: 1 minute at 94°C, 1 minute at 67°C, and I minute at 72°C with a final 5 minute extension at 72°C. Amplification was confirmed on a 2% agarose gel, and the samples were sequenced by the dye terminator method in both the forward and reverse directions. Sequencing results were manually evaluated at the SNP location.

*Study Design and End Points.* One objective of this study was to determine the relationship between genotype and relapse-free time, disease-free survival, and overall survival. Another objective was to determine whether the incidence of hot flashes differed with respect to genotype.

Relapse-free (RF) time was defined as the time from randomization to documentation of a breast event where a breast event is any recurrence (local, regional, or distant) of breast cancer or the documentation of contralateral breast cancer (including ductal carcinoma *in situ*). When estimating the distribution of RF time, patients who developed a non-breast second primary cancer (other than squamous or basal cell carcinoma of the skin, carcinoma *in situ* of the cervix, or lobular carcinoma *in situ* of the breast) prior to the diagnosis of a breast event were censored on the day their second primary was diagnosed. Patients (alive or dead) without a breast recurrence, contralateral breast cancer or a second non-breast primary cancer were censored at the date of their last disease evaluation.

Disease-free survival (DFS) was defined as the time from randomization to documentation of the first of the following events: any recurrence (local, regional or distant) of breast cancer, the documentation of contralateral breast cancer, or death due to any cause. Patients who were alive without a breast recurrence, contralateral breast cancer, or a second non-breast primary cancer were censored at the date of their last disease evaluation. Patients who developed a second primary (prior to breast recurrence or a contralateral breast cancer) were censored on the date the second primary was diagnosed.

Overall survival (OS) was estimated as the time from registration to death due to any cause.

The distributions of RF time, DFS, and OS were estimated overall using the Kaplan-Meier method. For each patient and pathologic factor and each clinical outcome, the following approach was used to assess the strength of the relationship between the factor and the outcome and to assess the proportional hazard assumption. A log rank test was used to assess the association between the factor and the outcome of interest. To examine whether the proportional hazards assumption was appropriate, a plot of the log of the stratum-specific cumulative hazard functions of that factor (determined using Kaplan Meier estimates) against time was constructed and examined for lack of parallelism. Also, a Cox model with the factor of interest and the interaction term composed of the factor with log (time) was fit to the data and then the significance of the interaction term was assessed to evaluate whether the hazard depended upon time.

Cox multivariate modeling was then used to determine which subset of patient and pathologic characteristics from among age (< 65 years vs. > 65), extent of surgery (mastectomy vs. breast conservation therapy), primary tumor size (< 3 cm vs. >3 cm), axillary lymph node status (positive vs. negative), and ER status (10-49 fmol vs. 50 fmol or greater vs. positive by immunohistochemical assay) was significantly associated with each clinical endpoint. Model assumptions and adequacy of fit were examined using residual plots such as: dfbeta statistic (a transform of the score residual) versus patient rank order of study entry, Martingale residuals versus linear predictor scores, and deviance residual versus linear predictor scores.

The potential prognostic value of each genotype in terms of RF time, DFS, and OS was expressed using three categories: no variant alleles, one variant allele, and two variant alleles. The log-rank test and the generalized Wilcoxon test were then used to assess whether RF time, DFS, or OS differed with respect to genotype. For each endpoint and each genotype, the Cox model that was previously found to provide the best fit from among those containing a subset of patient and pathologic characteristics was expanded to include the genotype represented in terms of two indicator variables. The likelihood ratio tests were then used to ascertain whether one or both of the indicator variables made a significant contribution to the model. For all CYP2D6*4 analyses, the "wild-type" allele refers to the absence of the *4 allele. Hot flashes were graded using the NCCTG supplement to the NCI common toxicity criteria (version 1) as follows: O-none or no change, 1-mild, 2-moderate, or 3-severe. The Wilcoxon rank sum test was used to assess whether the severity of hot flashes (0-3) differed with respect to genotype, and the one-sided Fisher's exact test was used to assess whether the proportion of women with moderate or severe hot flashes was smaller for those with the CYP2D6*4/*4 genotype than those without the CYP2D6*4/*4 genotype.

### Results

*Characteristics of the Patients.* Of the 256 women enrolled to the tamoxifen only arm, 213 paraffin-embedded tumor and 10 normal tissue blocks were available for DNA extraction. Table 4 presents the pre-registration characteristics of the 256 eligible patients randomized to the tamoxifen only arm that did and did not have a specimen from their primary surgery available for genotyping. The overall patient characteristics were similar, although a higher percentage of patients with available tissue had a tumor size greater than 3 cm (22%) compared with the group without tissue available (15%).

**Table 4: Pre-registration characteristics of the patients randomized to the tamoxifen arm that did and did not have paraffin embedded tumor tissue available from their primary breast surgery. Additionally, the patient characteristics of the CYP2D6*4/*4 group are shown.**

| | Women with paraffin tissue | Women without paraffin tissue | CYP2D6*4/*4 genotype |
|---|---|---|---|
| | (n=223) | (n=33) | (n=13) |
| Operative procedure | | | |
| Mastectomy | 83% | 73% | 92% |
| Breast conservation | 17% | 27% | 8% |
| Prior Hysterectomy | 42% | 45% | 40% |
| Prior BSO | 25% | 21% | 23% |
| Exogenous Estrogens | 16% | 15% | 0% |
| Number of Positive Nodes | | | |
| 0 | 62% | 64% | 31% |
| 1-3 | 26% | 15% | 54% |
| 4-9 | 7% | 15% | 8% |
| 10+ | 4% | 6% | 8% |
| Tumor Size ≥ 3 cm | 22% | 15% | 38% |
| ER status | | | |
| 10-49 fmols | 21% | 15% | 15% |
| ≥ 50 fmols | 67% | 61% | 62% |
| Positive | 12% | 24% | 23% |
| Median age (range) | 68 (42-87) | 69 (48-83) | 73 (56-87) |
| Caucasian | 92% | 91% | 100% |

For the group of 223 patients whose paraffin sample was available, the first documented event was as follows: local, regional or distant breast recurrence (43 patients), contralateral breast cancer (12 patients), a second non-breast primary cancer (16 patients), and death without a breast recurrence or second primary cancer (40 patients). At last follow-up, 112 women were alive without evidence of a breast event or second primary, 25 were alive following a breast event or second primary cancer, 33 died with disease recurrence, 13 died having developed a second primary cancer, 32 died of other causes, and 8 died of unknown causes. The Kaplan-Meier estimates for the 10 year RF time, DFS and OS were as follows: 75.0% (95% CI: 69.1-81.4%), 61.0% (54.7-68.0%), and 68.4% (62.5-74.9%). The median length of follow-up among the 137 patients still alive was 11.4 years (range: 5.7-14.1 yrs).

For the clinical endpoints of RF time, DFS, and OS, Cox modeling demonstrated that positive nodes and tumor size greater than 3 cm were significantly associated with decreased RF time, DFS, and OS.

*Genotype and allele frequency.* The CYP2D6 (*4 and *6) and CYP3A5 (*3) alleles were successfully amplified in 190, 194, and 205 patients, respectively, and their allelic frequencies are shown in Table 5. No CYP2D6 (*6) variants were detected. The genotype and allelic frequencies for each variant were similar to published reports in a predominantly Caucasian population.

**Table 5: Genotype and allele frequencies (q) for CYP2D6 (*4), and CYP3A5*3. No CYP2D6 (*6) variants were observed.**

| | | |
|---|---|---|
| CYP2D6 (*4) n=190 | | q=0.17 |
| Wt/Wt | 137 (72.1%) | |
| Wt/*4 | 40(21.1%) | |
| *4/*4 | 13(6.8%) | |
| CYP3A5 (*3) n=205 | | q=0.088 |
| A/A | 6 (2.9%) | |
| A/G | 24(11.7%) | |
| G/G | 175(85.4%) | |

### Clinical Outcome by Genotype.

### A. CYP2D6*4

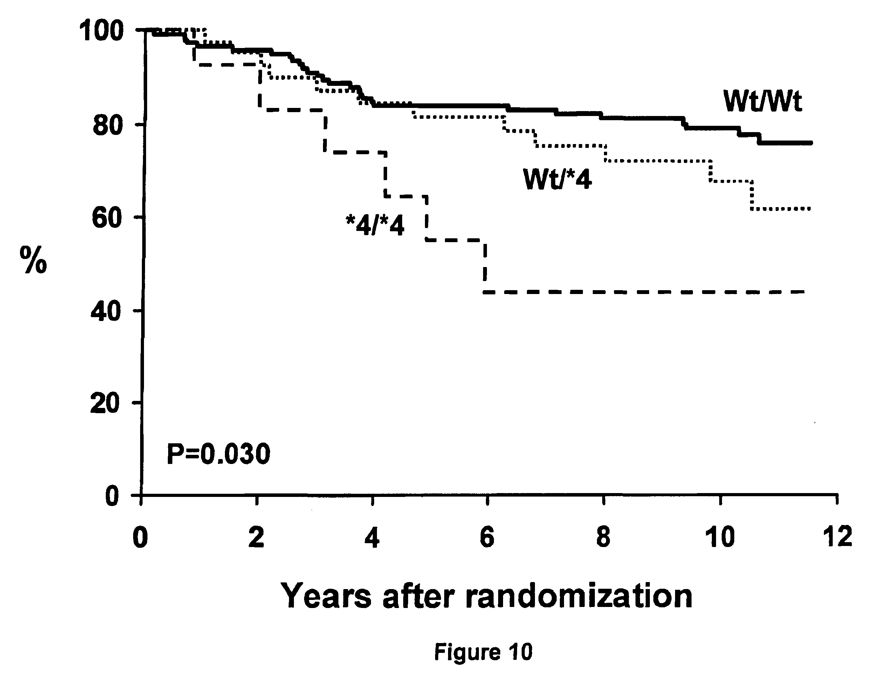

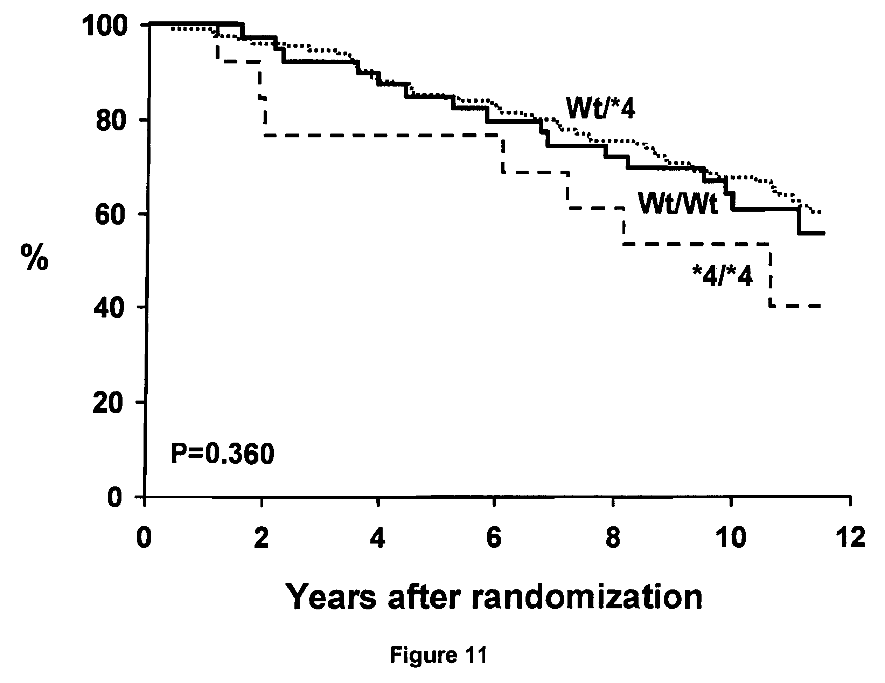

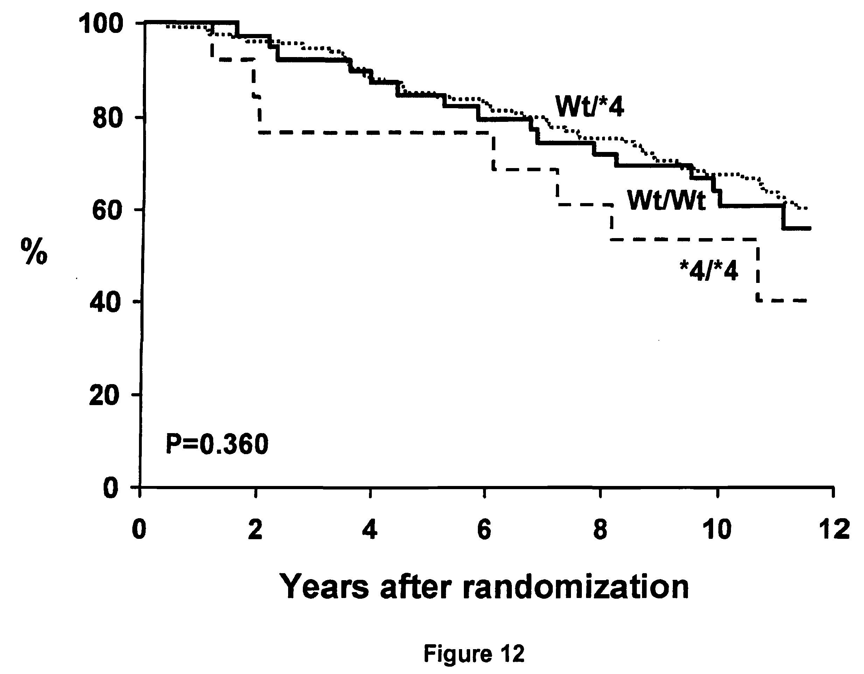

Patient characteristics for women with the CYP2D6*4/*4 genotype are presented in Table 4. A greater proportion of women with the CYP2D6*4/*4 genotype had node-positive disease. Women with the CYP2D6 *4/*4 genotype (n=13) had significantly worse RF time and DFS, but not OS, compared with either the *4/wt (n=40) or the wt/wt genotype (n=137; log rank p=0.030, p=0.020, and p=0.360, respectively; Figures 10-12). Cox proportional hazard modeling demonstrated that nodal status and tumor size were significantly associated with RF time, DFS, and OS. Once nodal status and tumor size were accounted for, women with the CYP2D6 *4/*4 genotype still tended to have worse RF time and DFS, but not OS, compared with patients with one or no variant alleles. Table 6 shows the unadjusted and adjusted hazard ratios comparing patients having the CYP2D6*4/*4 genotype with patients having the wt/wt or *4/wt genotypes.

**Table 6: Unadjusted and adjusted hazard ratios and corresponding 95% confidence intervals and p values comparing patients having the CYP2D6*4/*4 genotype with patients having the WT/WT or *4/WT genotypes.**

| Hazard Ratios for CYP2D6*4: *4/*4 relative to*4/WT and WT/WT | | | | |
|---|---|---|---|---|
| | Unadjusted | | Adjusted* | |
| | Hazard Ratio (95% CI) | P value | Hazard Ratio (95% CI) | P value |
| Relapse-free time | 2.71 (1.15-6.41) | 0.023 | 1.85(0.76-4.52) | 0.176 |
| Disease-free Survival | 2.44 (1.22-4.90) | 0.012 | 1.86 (0.91-3.82) | 0.089 |
| Overall Survival | 1.73 (0.79-3.76) | 0.169 | 1.12 (050-2.50) | 0.780 |

| | | | | |
|---|---|---|---|---|
| *A Cox model including nodal status and tumor size was used to estimate the adjusted hazard ratios | | | | |

### B. CYP3A5 (*3)

Neither RF time, DFS, nor OS was found to differ in terms of CYP3A5 *3 genotype (A/A vs. A/G vs. G/G: log-rank p-value=0.854, 0.937, and 0.950 respectively).

*Hot Flashes.* Among the 223 patients, a total of 61% (n = 136) reported having hot flashes with 40% (n = 90) reporting mild (grade 1), 15% (n = 34) reporting moderate (grade 2), and 5% (n = 12) reporting severe (grade 3) hot flashes. There were no differences in hot flash severity with respect to CYP3A5. However, for CYP2D6*4, none (0/13) of the women with the CYP2D6 *4/*4 genotype had moderate or severe hot flashes compared with 20% (36/177) for patients with either the *4/WT or WT/WT genotypes (one-sided p=0.06) (Table 7).

**Table 7: Incidence of moderate (grade 2) or severe (grade 3) hot flashes within genotype subgroups.**

| Genotype | % of patients who developed moderate or severe hot flashes |
|---|---|
| CYP3A5 (*3) [n=205] | |
| G/G | 37/175 (21%) |
| G/A | 6/24 (25%) |
| A/A | 1/6(17%) |
| CYP2D6 (*4) [n=190] | |
| *4/*4 | 0/13 (0%) |
| *4/Wt | 9/40 (23%) |
| Wt/Wt | 27/137 (20%) |

*Tumor and germline genotype concordance.* A total of 17 living women submitted a buccal specimen. For CYP2D6*4, there were 15 patients with concomitant tumor and buccal genotype, and the concordance between tumor and buccal genotype was 100% (15/15). Similarly for CYP3A5*3, there were 13 patients with concomitant tumor and buccal genotype and the concordance rate was 100% (13/13).

The results provided herein demonstrate that women homozygous for the most common allele associated with the CYP2D6 poor metabolizer (PM) phenotype, CYP2D6 *4, tend to have worse RF time (HR 1.85, p=0.176) and DFS (HR 1.86, p=0.089), once nodal status and tumor size were accounted for. The biologic importance of a CYP2D6 genotype was further supported by the finding that none of the women with the *4/*4 genotype experienced moderate or severe hot flashes compared with 20% of the women with either the *4/WT or WT/WT genotypes. By obtaining germline CYP2D6*4 genotype from living patients, 100% concordance between tumor and buccal genotype was demonstrated, suggesting that tumor CYP2D6*4 genotype is an accurate means by which to obtain CYP2D6*4 germline status.

In addition, the results provided herein suggest that CYP2D6 genetic variation is a determinant of tamoxifen effect and that lower or absent CYP2D6 activity may increase the risk of tamoxifen treatment failure. These findings can be confirmed in a larger prospective tamoxifen study in which a greater number of alleles corresponding to the CYP2D6 metabolizer status are evaluated.

### Example 3 - Assessing breast cancer outcomes based on CYP2D6 genotype and the use of CYP2D6 inhibitors

*Patients:* The North Central Cancer Treatment Group (NCCTG) conducted a randomized phase III clinical trial in postmenopausal women with resected ER positive breast cancer to assess the value of adding the androgen fluoxymesterone, for one year, to the standard five years of tamoxifen adjuvant therapy (NCCTG 89-30-52). For this trial, ER-positive was defined as ≥10 fmol ER polypeptide / mg cytosol protein in resected breast tissue by a standard biochemical assay or positive results for ER polypeptide expression in resected breast tissue by an immunohistochemical assay. The details of the clinical trial, including the eligibility requirements, are provided elsewhere (Ingle et al., North Central Cancer Treatment Group Trial 89-30-52, Breast Cancer Res. Treat. 2006)).

Using the 256 eligible patients randomized to the tamoxifen-only arm, the associations of CYP3A5 (*3) and CYP2D6 genetic variation (*4 and *6) with the outcomes of breast cancer relapse and death were determined as described herein. Paraffin-embedded tissue blocks were available in 223 patients, and CYP2D6*4 genotype was determined in 190 patients. In this example, the role of CYP2D6 inhibitors in patients randomized to the tamoxifen only arm was evaluated.

*Chart Review:* Patient charts were reviewed at each randomizing site to determine whether the following known inhibitors of the CYP2D6 enzyme system were co-administered during the five years that tamoxifen was prescribed: potent inhibitors (e.g., fluoxetine and paroxetine) or moderate inhibitors (e.g., sertraline, cimetidine, amiodarone, doxepin, ticlopidine, and haloperidol; Jin et al., J. Natl. Cancer Inst., 97(1):30-9 (2005); Crewe et al., Br. J. Clin. Pharmacol., 34(3):262-5 (1992); Martinez et al., Clin. Phamacol. Ther., 65(4):369-76 (1999); Funck-Brentano et al., Clin. Pharmacol. Ther., 50(3):259-66 (1991); Ohyama et al., Br. J. Clin. Pharmacol., 49(3):244-53 (2000); Szewczuk-Boguslawska et al., Pol. J. Pharmacol., 56(4):491-4 (2004); Ko et al., Br. J. Clin. Pharmacol., 49(4):343-51 (2000); and Shin et al., Br. J. Clin. Pharmacol., 51(1):45-52 (2001)). The length of time patients were co-prescribed CYP2D6 inhibitors and tamoxifen was recorded as follows: less than I year, 1-2 years, 2-3 years, 3-4 years, and 4-5 years.

*CYP2D6 Metabolizer Status:* Using the available *in vivo* data regarding the combined effect of CYP2D6 genotype and CYP2D6 inhibitors on endoxifen plasma concentrations (Jin et al., J. Natl. Cancer Inst., 97(1):30-9 (2005)), a CYP2D6 metabolizer status was assigned based on CYP2D6 *4 genotype and the co-administration (yes/no) of a CYP2D6 inhibitor during the five years that tamoxifen was prescribed. Extensive metabolizers (EM) were those individuals without a CYP2D6 *4 allele (Wt/Wt) and no inhibitor. Intermediate metabolizers (IM) were defined as (1) Wt/Wt genotype with co-administration of a moderate inhibitor or (2) heterozygous for the *4 allele (*4/Wt) and no inhibitor. Poor metabolizers (PM) were women (1) homozygous for the *4 allele (*4/*4), (2) *4/Wt and co-administration of a moderate or potent inhibitor, or (3) Wt/Wt and co-administration of a potent inhibitor.

*Study Design and End Points:* The primary objectives were to determine the effect of CYP2D6 metabolizer status on the outcomes of time to breast cancer recurrence (TTBR), relapse-free survival (RFS), disease-free survival (DFS), and overall survival (OS).

TTBR was defined as the time from randomization to documentation of a breast event where a breast event is any recurrence (local, regional, or distant) of breast cancer or the documentation of contralateral breast cancer (including ductal carcinoma *in situ*). When estimating TTBR, patients who developed a non-breast second primary cancer (other than squamous or basal cell carcinoma of the skin, carcinoma *in situ* of the cervix, or lobular carcinoma *in situ* of the breast) prior to the diagnosis of a breast event were censored on the day their second primary was diagnosed. Patients without a breast recurrence, contralateral breast cancer, or a second non-breast primary cancer were censored at the date of their last disease evaluation. RFS was defined as the time from randomization to documentation of the first of the following events: any recurrence (local, regional, or distant) of breast cancer, a contralateral breast cancer, or death. When estimating the distribution of RFS, patients who developed a non-breast second primary cancer (other than squamous or basal cell carcinoma of the skin, carcinoma *in situ* of the cervix, or lobular carcinoma *in situ* of the breast) prior to the diagnosis of a breast event were censored on the day their second primary was diagnosed. Patients who were alive without a breast recurrence, contralateral breast cancer, or a second non-breast primary cancer were censored at the date of their last disease evaluation. DFS was defined as the time from randomization to documentation of the first of the following events: any recurrence (local, regional, or distant) of breast cancer, a contralateral breast cancer, a second primary cancer, or death due to any cause. Patients who were alive without any of these events were censored at the date of their last disease evaluation. OS was estimated as the time from registration to death due to any cause.

The overall distributions of TTBR, RFS, DFS, and OS were estimated using the Kaplan-Meier method. Log rank tests and univariate Cox proportional hazard models were used to assess whether the endpoint differed with respect to any one of the following factors: age 65 years or greater (yes vs. no), extent of surgery (mastectomy vs. breast conserving), ER status as recorded at time of entry into the trial (10-49 fmols vs. ≥50 fmols vs. positive by immunohistochemistry), positive nodes (yes/no), tumor size 3 cm or greater (yes vs. no), Nottingham grade (3 vs. I or 2), Her2 expression (3+ vs. 0, 1+, or 2+), and prior exposure to exogenous estrogens (yes vs. no). For each clinical outcome, multivariate Cox proportional hazard modeling was performed to obtain a subset of the potential prognostic factors which provided an adequate fit to the data. Residual plots were examined. Cox multivariate modeling was then used to determine whether metabolizer status made a significant contribution to the previous established model for that clinical endpoint. The RFS hazard function was estimated using a kernel based approach with the global bandwidth selection algorithm and boundary kernel formulation proposed by Muller and Wang (Muller and Wang, Biometrics, 50(1):61-76 (1994) and Hess et al., Stat. Med., 18(22):3075-88 (1999)).

### Results

CYP2D6*4 genotype was obtained in 190 of the 256 eligible patients enrolled where CYP2D6*4/*4 genotype frequency was 6.8%. Medical charts were available for review for 225 patients. Thirteen patients (6%) were co-prescribed a CYP2D6 inhibitor (potent (n=3), moderate (n=10)) during the five years of tamoxifen. The median duration of co-administration of tamoxifen and the CYP2D6 inhibitor was 2-3 years in 11 patients. In two patients, the duration of therapy was unknown.

Metabolizer status was determined in 171 patients using CYP2D6*4 genotype and CYP2D6 inhibitor data (Table 8). Of the 124 patients who did not carry a *4 allele (Wt/Wt), 115 were considered EM (no inhibitors), eight were 1M (moderate inhibitor), and one was a PM (potent inhibitor). Of the 33 patients heterozygous for the *4 allele (*4/Wt), 32 were classified as IM (no inhibitor), and one was a PM (potent inhibitor). Thirteen patients homozygous for the *4 allele (*4/*4) were classified as PM. One additional patient with unknown genotype was classified as a PM based on the co-administration of a potent inhibitor. Two patients who were co-prescribed moderate inhibitors were not classified because of unknown genotype. The patient characteristics of these 171 patients are listed in Table 9 and were similar to the eligible patients whose metabolizer status could not be determined (n=85).

**Table 8: Metabolizer status according to CYP2D6*4 genotype and co-administration of a CYP2D6 inhibitor.**

| Metabolism status | Genotype | Moderate inhibitor | Potent inhibitor | N=171 |
|---|---|---|---|---|
| Extensive | Wt/Wt | no | no | 115(67.3%) |
| Intermediate | Wt/Wt | yes | no | 8 (4.7%) |
| | Wt/*4 | no | no | 32 (18.7%) |
| | unknown | no | yes | 1(0.6%) |
| | *4/*4 | unknown | unknown | 2(1.2%) |
| Poor | *4/*4 | no | no | 11(6.4%) |
| | Wt/*4 | no | yes | 1 (0.6%) |
| | Wt / Wt | no | yes | 1 (0.6%) |

**Table 9: Patient characteristics comparing those with known versus unknown CYP2D6 metabolizer status.**

| Patient Characteristics | Metabolizer status known (n=171) | Metabolizer status unknown (n=85) |
|---|---|---|
| Median Age (range) | 68 (42-84) | 69 (47-87) |
| Operative Procedure | | |
| Mastectomy | 83% | 79% |
| Breast conserving | 17% | 21% |
| Tumor size | | |
| < 3 cm | 77% | 82% |
| ≥ 3 cm | 23% | 18% |
| Tumor grade | | |
| 1 | 24% | 15% |
| 2 | 54% | 31% |
| 3 | 15% | 12% |
| unknown | 7% | 42% |
| Number of positive nodes | | |
| 0 | 64% | 59% |
| 1-3 | 26% | 24% |
| 4-9 | 6% | 12% |
| 10+ | 4% | 6% |
| Estrogen Receptor (at randomization) | | |
| 10-49 fmols | 21% | 18% |
| ≥ 50 fmols | 66% | 67% |
| positive | 13% | 15% |
| Her-2 | | |
| 0 | 10% | 9% |
| 1 | 33% | 22% |
| 2 | 33% | 18% |
| 3 | 19% | 9% |
| unknown | 5% | 41% |
| Nottingham tumor Grade | | |
| Grade 1 | 24% | 15% |
| Grade 2 | 54% | 31% |
| Grade 3 | 15% | 12% |
| Unknown | 7% | 42% |

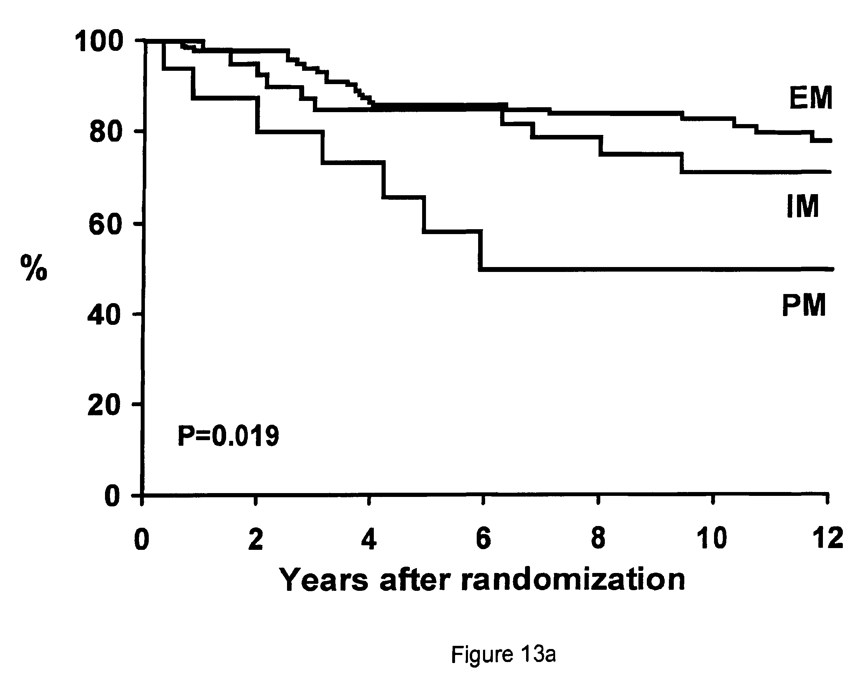

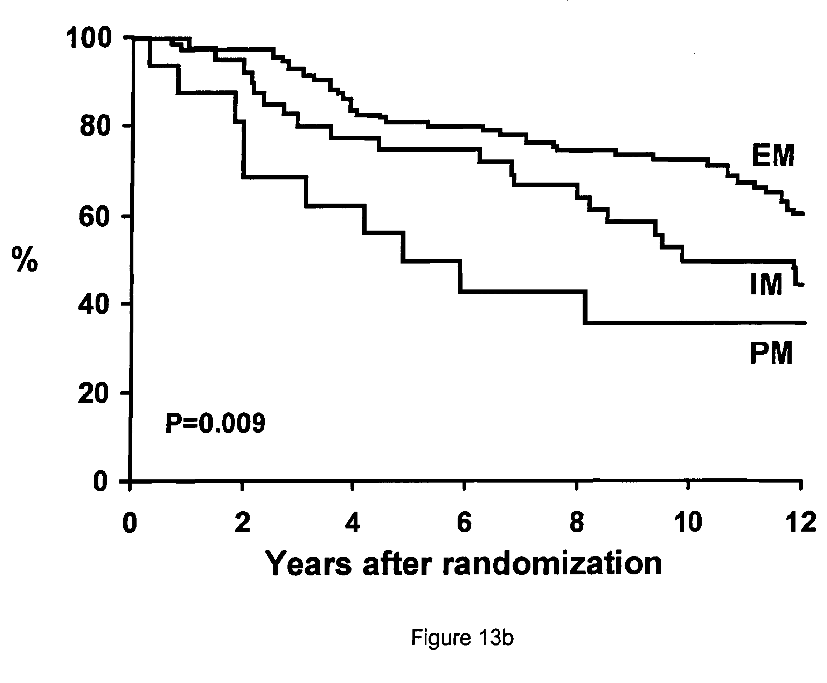

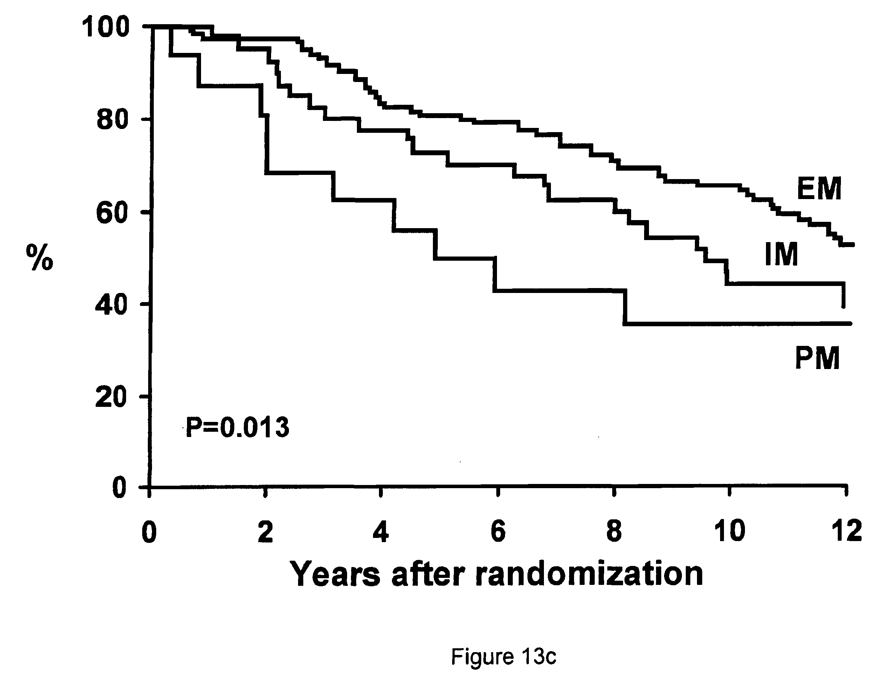

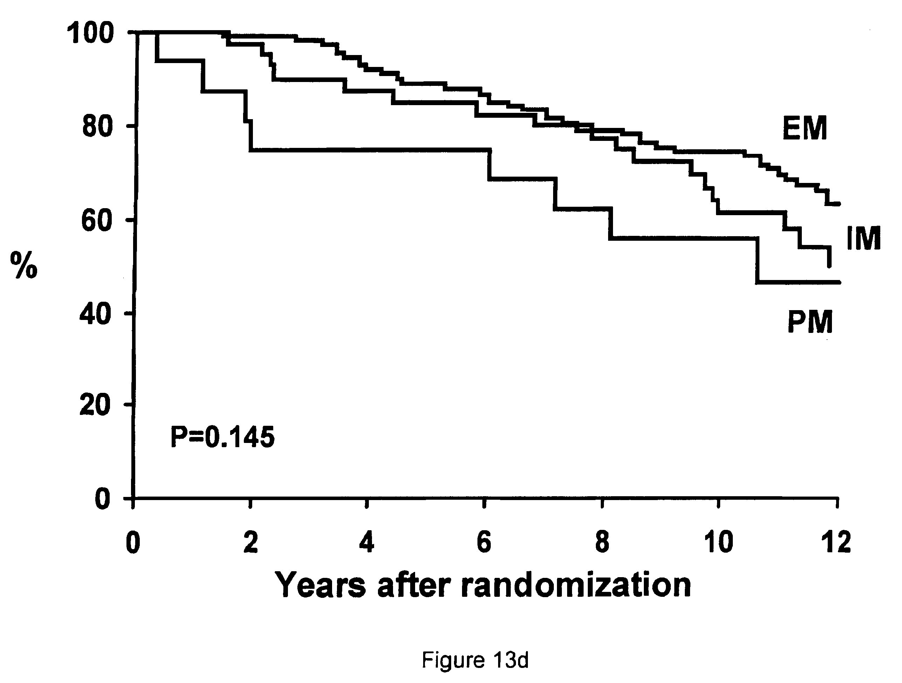

*Clinical Outcome by Metabolizer Status:* A Cox model was used to assess whether clinical benefit was significantly decreased for IM or PM relative to the EM. Poor metabolizers had significantly shorter TTBR (p=0.007), RFS (p=0.005), DFS (p=0.008), and tended to have worse OS (p=0.077) than EM. Intermediate metabolizers did not have shorter TTBR (p=0.338), but tended to have worse RFS (p=0.075) and DFS (p=0.097) compared to extensive metabolizers (Table 10). The Kaplan Meier curves for TTBR, RFS, DFS, and OS by metabolizer class were prepared (Figure 13). The two year RFS rates by metabolizer status were 97.5%, 92%, and 68% for EM, IM, and PM, respectively (Figure 13b).

**Table 10: Cox proportional hazards model assessing clinical outcome according to metabolizer status**

| Outcome | Estimated unadjusted hazard ratio relative to extensive metabolizers (Corresponding 95% Confidence Interval) | p-value |
|---|---|---|
| Time to Breast recurrence | 3.2 (1.37-7.55) | 0.007 |
| PM | 1.4 (0.68-3.05) | 0.3375 |
| IM | | |
| Relapse-free survival | | |
| PM | 2.69 (1.34-5.37) | 0.005 |
| IM | 1.63 (0.95-2.78) | 0.075 |
| Disease-free survival | | |
| PM | 2.44 (1.27-4.69) | 0.008 |
| IM | 1.52 (0.93-2.49) | 0.097 |
| Overall survival | | |
| PM | 2.0 (0.92-4.17) | 0.077 |
| IM | 1.40 (0.80-2.43) | 0.240 |

Cox proportional hazard modeling demonstrated that tumor size was significantly associated with TTBR. After adjusting for tumor size, TTBR relative to EM was significantly shorter among PM (HR 2.98, p=0.012) but not among IM (HR 1.40, p=0.376). Tumor size, nodal status, and tumor grade were significantly associated with RFS, DFS, and OS. Once nodal status, tumor grade, and size were accounted for, RFS and DFS times relative to EM were significantly decreased for PM (HR 2.37, p=0.020 & HR 2.12, p=0.031, respectively) and tended to be decreased for IM (HR 1.76, p=0.047 & HR 1.65, p=0.054, respectively; Table 11).

**Table 11: Results of Cox modeling of time to breast recurrence (TTBR), relapse-free survival (RFS), disease-free survival (DFS), and overall survival (OS).**

| Endpoint | Size (< 3cm vs. ≥ 3cm) | Grade (1, 2 vs. 3) | Node positive Disease (yes vs. no) | CYP2D6 poor metabolizcr (yes vs. no) | CYP2D6 intermediate metabolizer (yes vs. no) |
|---|---|---|---|---|---|
| TTBR | 2.60 (1.36-4.97) | | | 2.98 (1.27-7.02) | 1.40 (0.66-2.96) |
| RFS | 1.75 (1.04-2.94) | 2.04 (1.12-3.72) | 1.61 (0.98-2.65) | 2.37 (1.15-4.89) | 1.76 (1.01-3.07) |
| DFS | 2.01 (1.26-3.21) | 1.97 (1.13-3.44) | 1.66 (1.05-2.63) | 2.12 (1.07-4.19) | 1.65 (0.99-2.76) |

The results provided herein demonstrate that CYP2D6 metabolizer status (PM) is an independent predictor of breast cancer outcome in postmenopausal women treated with tamoxifen. These results also demonstrate that even partial reduction in the activity of CYP2D6 (IM) is associated with worse relapse-free survival (HR 1.76, p=0.047) in the multivariate analyses. The results provided herein can have practice implications for hormonal therapy of breast cancer. First, these results demonstrate that physicians and patients should consider CYP2D6 genotyping prior to initiating tamoxifen therapy for the purpose of excluding CYP2D6 PM. The second practice implication pertains to the co-administration of drugs which decrease CYP2D6 activity. Antidepressant drugs, such as the selective serotonin reuptake inhibitors and the serotonin and norepinephrine reuptake inhibitors, are commonly administered with tamoxifen in breast cancer patients to treat depression or alleviate hot flashes and have been considered an important advance in the nonhormonal therapy of hot flashes. The results provided herein support the hypothesis that co-administration of CYP2D6 inhibitors decreases the effectiveness of tamoxifen by inhibiting the metabolic activation of tamoxifen. Based on these data, physicians and patients should exercise great caution when prescribing any drug known to inhibit CYP2D6 in the presence of tamoxifen.

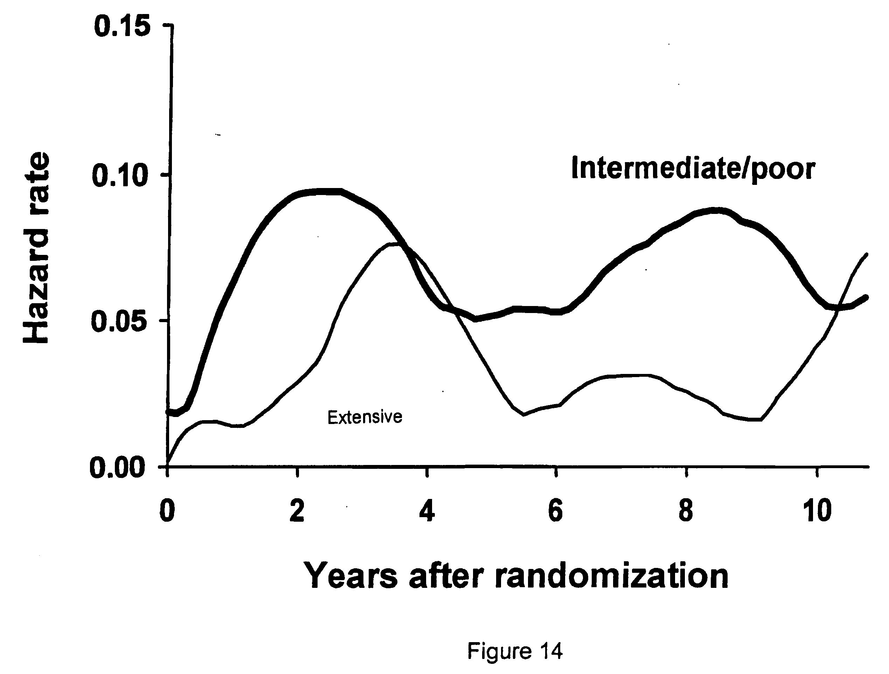

When comparing instantaneous hazard rates for RFS by metabolizer status (Figure 14), the data demonstrate an immediate broad peak in the RFS hazard rate for PM and IM. In contrast, the hazard rate in EM was reduced and did not peak until nearly the 4^{th} year. These findings suggest that the peak in the hazard rate for recurrence seen following the initiation of tamoxifen is in part due to impaired CYP2D6 metabolism, and switching patients to an aromatase inhibitor after 2-3 years of tamoxifen may simply rescue some patients with decreased ability to activate tamoxifen. Because the administration of an aromatase inhibitor following five years of tamoxifen and after 2-3 years of tamoxifen significantly prolongs disease free and overall survival, CYP2D6 status may identify those patients (EM and UM) most suitable for sequencing of hormonal therapy.

In summary, by accounting for exposure to commonly administered medications that inhibit CYP2D6 activity, CYP2D6 status was identified as being an independent predictor of tamoxifen efficacy. These results suggest that CYP2D6 may provide a means by which the hormonal therapy of breast cancer can be individualized. Physicians and patients should be aware that the co-prescription of moderate or potent CYP2D6 inhibitors may increase the risk of breast cancer relapse by inhibiting the metabolic activation of tamoxifen.

### Example 4 - Assessing breast cancer outcomes

The following was performed to evaluate using the HOXB13:IL17BR expression ratio in combination with CYP2D6 genotype and/or the patient's history of taking a CYP2D6 inhibitor while taking tamoxifen (the Goetz index) to assess relapse and survival in a prospective adjuvant tamoxifen trial.

In lymph node negative, ER positive breast cancer patients who were treated with tamoxifen on a prospective NCCTG clinical trial (NCCTG 89-30-52), CYP2D6*4 genotype was determined as described herein. Medical records were reviewed at each randomizing site to determine whether potent (e.g., fluoxetine and paroxetine) or moderate (e.g., sertraline, cimetidine, amiodarone, doxepin, ticlopidine, and haloperidol) CYP2D6 inhibitors were co-prescribed with tamoxifen. Patients were grouped by CYP2D6 metabolizer status (poor, intermediate, or extensive) based on the presence of none, one, or two CYP2D6 *4 alleles and whether they were co-administered potent or moderate CYP2D6 inhibitors. The HOXB13:IL17BR expression ratio was determined as described herein. Patients were divided into high risk (high HOXB13:IL17BR expression ratio and CYP2D6 poor metabolizer), low risk (low HOXB 13:IL 17BR expression ratio and CYP2D6 extensive metabolizer), or intermediate risk (all others). The association between Goetz index and clinical outcome was determined using the log-rank test.

The Goetz index was determined in 110 women with ER +, lymph node negative breast cancer treated with tamoxifen (Table 12). Forty-six patients (42%) were low risk, fifty patients (45%) were intermediate risk, and 14 (13%) were high risk. Patients who were high risk by the Goetz index had worse relapse free survival (p=0.007), and overall survival (p=0.005) compared with either intermediate or good risk patients. The 9 year RFS rate according to the Goetz index was as follows: low (87%), intermediate (65%), and high (46%). Among these 110 patients with node-negative disease, there is evidence to suggest that time to breast recurrence (p=0.075), RFS (p=0.007), DFS (p=0.001), or OS (p=0.005) differ with respect to risk profile.

The results provided herein demonstrate that the Goetz index can be used to identify patients with low risk ER +, lymph node negative breast cancer who can be treated with tamoxifen monotherapy for five years. Additional therapies should be studied in patients identified as either intermediate or high risk by means of the Goetz index.

**Table 12. Results using Goetz index.**

| Risk Profile | HOXB13:IL-17BR ratio | CYP2D6 Genotype | Medication history | N=110 |
|---|---|---|---|---|
| Low | < -1.339 | Wt/Wt | no | 46(41.8%) |
| | ≥ -1.339 | Wt / Wt | no | 32(29.1%) |
| | | unknown | yes | 2(1.8%) |
| | | Wt/*4 | yes | 1(0.9%) |
| Intermediate | < -1.339 | Wt/Wt | yes | 5(4.6%) |
| | | *4/*4 | no | 1(0.9%) |
| | | Wt/*4 | no | 9(8.2%) |
| | | Wt/Wt | yes | 1(0.9%) |
| High | ≥ -1.339 | *4/*4 | no | 2(1.8%) |
| | | Wt/*4 | no | 11(10.0%) |

Additional data from this study are presented below.

**Table 13. CYP2D6 Metabolism Phenotype.**

| CYP2D6 Metabolism Phenotype | CYP2D6*4 Genotype | CYP2D6 Inhibitor | n=110 |
|---|---|---|---|
| Extensive | Wt/Wt | no | 78(71%) |
| Decreased | Wt/*4 | no | 20(18%) |
| | Wt/*4 | yes | 1(1%) |
| | *4/*4 | no | 3 (3 %) |
| | Wt/Wt | yes | 6(5%) |
| | unknown | yes | 2(2%) |

**Table 14. The 5 year DFS rate according to the combined index of HOXB13/IL17BR and CYP2D6.**

| Risk Profile | 2-Gene Ratio | CYP2D6 | N=110 | 5-year DFS |
|---|---|---|---|---|
| 1 | <-1.339 (low risk) | Extensive (low risk) | 46 | 94% |
| 2 | <-1.339 (low risk) | Decreased (high risk) | 32 | 75% |
| 3 | >-1.339 (high risk) | Extensive (low risk) | 18 | 83% |
| 4 | >-1.339 (high risk) | Decreased (high risk) | 14 | 57% |

**Table 15. Results of Cox modeling of DFS and OS in Node-Negative Patients according to the combined index of HOXB13/IL17BR and CYP2D6 metabolism: Hazard Ratios (and corresponding 95% confidence intervals).**

| Factor | Clinical Outcome | |
|---|---|---|
| | DFS | OS |
| Tumor size | 2.77(1.5-5.12) | 2.61(1.30-5.25) |
| HOXB13/IL17BR and CYP2D6 index | 2.27 (1.20-4.29) | 2.51 (1.16-5.46) |

The results provided herein demonstrate that the Goetz index can be used to identify four risk groups: (1) extensive CYP2D6 metabolism, low HOXB13:IL17BR expression ratio, (2) decreased CYP2D6 metabolism, low HOXB13:IL17BR expression ratio, (3) extensive CYP2D6 metabolism, high HOXB13:IL17BR expression ratio, and (4) decreased CYP2D6 metabolism, high HOXB13:IL17BR expression ratio. The Goetz index, which combines the HOXB13:IL17BR expression ratio with the CYP2D6 genotype, is an independent predictor of DFS and OS, even after accounting for tumor size (Table 4).

## Claims

1. A method for assessing the likelihood of cancer relapse, wherein said method comprises:
(a) determining whether or not a breast cancer patient being treated with tamoxifen contains cancer tissue with a HOXB13:IL17BR expression ratio greater than -1.339,
(b) determining whether or not said breast cancer patient contains a CYP2D6 *4/WT or a CYP2D6 *4/*4 genotype, and
(c) determining whether or not said breast cancer patient is taking medication selected from the group consisting of fluoxetine, paroxetine sertraline, cimetidine, amiodarone, doxepin, ticlopidine, and haloperidol,
wherein the presence of said HOXB13:IL17BR expression ratio greater than -1.339, the presence of said genotype and taking said medication indicates that said patient is likely to experience cancer relapse.

2. The method of claim 1, wherein said breast cancer patient is a node-negative breast cancer patient.

3. The method of claim 1, wherein the absence of said HOXB13:IL17BR expression ratio greater than -1.339, the absence of said genotype, and not taking said medication indicates that said patient is likely to experience a relapse-free survival or a disease-free survival.

4. The method of claim 1, wherein determining whether or not said breast cancer patient contains cancer tissue with said HOXB13:IL17BR expression ratio greater than - 1.339 comprises using the HOXB13:IL-17BR calculation method.

5. A method for assessing the likelihood of cancer survival, wherein said method comprises:
(a) determining whether or not a breast cancer patient being treated with tamoxifen contains cancer tissue with a HOXB13:IL17BR expression ratio less than -1.339,
(b) determining whether or not said breast cancer patient contains a CYP2D6 *4/WT or a CYP2D6 *4/*4 genotype, and
(c) determining whether or not said breast cancer patient is taking medication selected from the group consisting of fluoxetine, paroxetine sertraline, cimetidine, amiodarone, doxepin, ticlopidine, and haloperidol,
wherein the presence of said HOXB13:IL17BR expression ratio less than -1.339, the absence of said genotype and not taking said medication indicates that said patient is likely to experience longer survival than a comparable breast cancer patient who contains cancer tissue with a HOXB13:IL17BR expression ratio greater than -1.339 who contains said genotype and who is taking said medication.

6. The method of claim 5, wherein said breast cancer patient is a node-negative breast cancer patient.

7. The method of claim 5, wherein the absence of said HOXB13:IL17BR expression ratio less than -1.339, the presence of said genotype and taking said medication indicates that said patient is likely to experience shorter survival than a comparable breast cancer patient who contains cancer tissue with a HOXB13:IL17BR expression ratio less than -1.339, who lacks said genotype and who is not taking said medication.

8. The method of claim 5, wherein determining whether or not said breast cancer patient contains cancer tissue with said HOXB13:IL17BR expression ratio less than -1.339 comprises using the HOXB13:IL-17BR calculation method.

9. A method for assessing the likelihood of cancer relapse, wherein said method comprises:
(a) determining whether or not a breast cancer patient taking tamoxifen contains cancer tissue with a HOXB13:IL17BR expression ratio greater than -1.339, and
(b) determining whether or not said breast cancer patient is taking medication selected from the group consisting of fluoxetine, paroxetine sertraline, cimetidine, amiodarone, doxepin, ticlopidine, and haloperidol, wherein said medication is being taken concurrently with said tamoxifen, wherein the presence of said HOXB13:IL17BR expression ratio greater than -1.339 and taking said medication indicates that said patient is likely to experience cancer relapse.

10. The method of claim 9, wherein said breast cancer patient is a node-negative breast cancer patient.

11. The method of claim 9, wherein the absence of said HOXB13:IL17BR expression ratio greater than -1.339 and not taking said medication indicates that said patient is likely to experience a relapse-free survival or a disease-free survival.

12. The method of claim 9, wherein determining whether or not said breast cancer patient taking tamoxifen contains cancer tissue with said HOXB13:IL17BR expression ratio greater than -1.339 comprises using the HOXB13:IL-17BR calculation method.

## Patentansprüche

1. Ein Verfahren zur Bewertung der Wahrscheinlichkeit eines Krebsrückfalls, wobei das Verfahren umfasst:
(a) Bestimmen, ob oder ob nicht ein Brustkrebspatient, der mit Tamoxifen behandelt wird, Krebsgewebe mit einem HOXB13:IL17BR Expressionsverhältnis von größer als - 1,339 enthält,
(b) Bestimmen, ob oder ob nicht der Brustkrebspatient einen CYP2D6 *4/WT oder einen CYP2D6 *4/*4 Genotyp enthält, und
(c) Bestimmen, ob oder ob nicht der Brustkrebspatient ein Medikament nimmt, das ausgewählt ist aus der Gruppe bestehend aus Fluoxetin, Paroxetin Sertralin, Cimetidin Amiodaron, Doxepin, Ticlopidin und Haloperidol,
wobei das Vorliegen des HOXB13:IL17BR Expressionsverhältnisses von größer als - 1,339, das Vorliegen des Genotyps und das Nehmen des Medikaments anzeigt, dass der Patient wahrscheinlich einen Krebsrückfall erlebt.

2. Das Verfahren von Anspruch 1, wobei der Brustkrebspatient ein nodal-negativer Brustkrebspatient ist.

3. Das Verfahren von Anspruch 1, wobei die Abwesenheit des HOXB13:IL17BR Expressionsverhältnisses von größer als -1,339, die Abwesenheit des Genotyps und das Nicht-Einnehmen des Medikaments anzeigt, dass der Patient wahrscheinlich ein rückfallfreies Überleben oder ein ktankheitsfreies Überleben erlebt.

4. Das Verfahren von Anspruch 1, wobei das Bestimmen, ob oder ob nicht der Brustkrebspatient Krebsgewebe mit dem HOXB13:IL17BR Expressionsverhältnis von größer als -1,339 enthält, die Verwendung des HOXB13:IL17BR Berechnungsverfahrens umfasst.

5. Ein Verfahren zur Bewertung der Wahrscheinlichkeit von Krebsüberleben, wobei das Verfahren umfasst:
(a) Bestimmen, ob oder ob nicht ein Brustkrebspatient, der mit Tamoxifen behandelt wird, Krebsgewebe mit einem HOXB13:IL17BR Expressionsverhältnis von weniger als -1,339 enthält,
(b) Bestimmen, ob oder ob nicht der Brustkrebspatient einen CYP2D6 *4/WT oder einen CYP2D6 *4/*4 Genotyp enthält, und
(c) Bestimmen, ob oder ob nicht der Brustkrebspatient ein Medikament nimmt, das ausgewählt ist aus der Gruppe bestehend aus Fluoxetin, Paroxetin Sertralin, Cimetidin Amiodaron, Doxepin, Ticlopidin und Haloperidol,
wobei das Vorliegen des HOXB13:IL17BR Expressionsverhältnisses von weniger als -1,339, die Abwesenheit des Genotyps und das Nicht-Einnehmen des Medikaments anzeigt, dass der Patient wahrscheinlich länger überlebt als ein vergleichbarer Brustkrebspatient, der Krebsgewebe mit einem HOXB13:IL17BR Expressionsverhältnis von größer als -1,339 enthält, der den Genotyp enthält und der das Medikament nimmt.

6. Das Verfahren von Anspruch 5, wobei der Brustkrebspatient ein nodal-negativer Brustkrebspatient ist.

7. Das Verfahren von Anspruch 5, wobei die Abwesenheit des HOXB13:IL17BR Expressionsverhältnisses von weniger als -1,339, das Vorliegen des Genotyps und das Nehmen des Medikaments anzeigt, dass der Patient wahrscheinlich kürzer überlebt als ein vergleichbarer Brustkrebspatient, der Krebsgewebe mit einem HOXB13:IL17BR Expressionsverhältnis von weniger als -1,339 enthält, der den Genotyp nicht hat und der das Medikament nicht nimmt.

8. Das Verfahren von Anspruch 5, wobei das Bestimmen, ob oder ob nicht der Brustkrebspatient Krebsgewebe mit dem HOXB13:IL17BR Expressionsverhältnis von weniger als -1,339 enthält, die Verwendung des HOXB13:IL17BR Berechnungsverfahrens umfasst.

9. Ein Verfahren zur Bewertung der Wahrscheinlichkeit eines Krebsrückfalls, wobei das Verfahren umfasst:
(a) Bestimmen, ob oder ob nicht ein Brustkrebspatient, der Tamoxifen nimmt, Krebsgewebe mit einem HOXB13:IL17BR Expressionsverhältnis von größer als -1,339 enthält, und
(b) Bestimmen, ob oder ob nicht der Brustkrebspatient ein Medikament nimmt, das ausgewählt ist aus der Gruppe bestehend aus Fluoxetin, Paroxetin Sertralin, Cimetidin Amiodaron, Doxepin, Ticlopidin und Haloperidol, wobei das Medikament gleichzeitig mit dem Tamoxifen genommen wird,
wobei das Vorliegen des HOXB13:IL17BR Expressionsverhältnisses von größer als - 1,339 und das Nehmen des Medikaments anzeigt, dass der Patient wahrscheinlich einen Krebsrückfall erlebt.

10. Das Verfahren von Anspruch 9, wobei der Brustkrebspatient ein nodal-negativer Brustkrebspatient ist.

11. Das Verfahren von Anspruch 9, wobei die Abwesenheit des HOXB13:IL17BR Expressionsverhältnisses von größer als -1,339 und das Nicht-Einnehmen des Medikaments anzeigt, dass der Patient wahrscheinlich ein rückfallfreies Überleben oder ein krankheitsfreies Überleben erlebt.

12. Das Verfahren von Anspruch 9, wobei das Bestimmen, ob oder ob nicht der Tamoxifen-nehmende Brustkrebspatient Krebsgewebe mit dem HOXB13:IL17BR Expressionsverhältnis von größer als -1,339 enthält, die Verwendung des HOXB13:IL17BR Berechnungsverfahrens umfasst.

## Revendications

1. Procédé d'évaluation de la probabilité d'une récidive de cancer, dans lequel ledit procédé comprend :
(a) la détermination de la présence ou non d'un rapport d'expression HOXB13 : IL17BR supérieur à -1,339 dans un tissu cancéreux d'une patiente atteinte d'un cancer du sein traitée avec du tamoxifène,
(b) la détermination de la présence ou non d'un génotype CYP2D6*4/WT ou CYP2D6*4/*4 chez ladite patiente atteinte d'un cancer du sein, et
(c) la détermination de la prise ou non d'un médicament choisi dans le groupe constitué par la fluoxétine, la paroxétine, la sertraline, la cimétidine, l'amiodarone, la doxépine, la ticlopidine et l'halopéridol par ladite patiente atteinte d'un cancer du sein,
dans lequel la présence dudit rapport d'expression HOXB13 : IL17BR supérieur à -1,339, la présence dudit génotype et la prise dudit médicament indiquent que ladite patiente est susceptible de subir une récidive de cancer.

2. Procédé selon la revendication 1, dans lequel ladite patiente atteinte d'un cancer du sein est une patiente atteinte d'un cancer du sein sans envahissement ganglionnaire.

3. Procédé selon la revendication 1, dans lequel l'absence dudit rapport d'expression HOXB13 : IL17BR supérieur à -1,339, l'absence dudit génotype et la non prise dudit médicament indiquent que ladite patiente est susceptible de survivre sans récidive ou de survivre sans maladie.

4. Procédé selon la revendication 1, dans lequel la détermination de la présence ou non dudit rapport d'expression HOXB13 : IL17BR supérieur à -1,339 dans un tissu cancéreux de ladite patiente atteinte d'un cancer du sein comprend l'utilisation de la méthode de calcul HOXB13 : IL-17BR.

5. Procédé d'évaluation de la probabilité de survie à un cancer, dans lequel ledit procédé comprend :
(a) la détermination de la présence ou non d'un rapport d'expression HOXB13 : IL17BR inférieur à -1,339 dans un tissu cancéreux d'une patiente atteinte d'un cancer du sein traitée avec du tamoxifène,
(b) la détermination de la présence ou non d'un génotype CYP2D6*4/WT ou CYP2D6*4/*4 chez ladite patiente atteinte d'un cancer du sein, et
(c) la détermination de la prise ou non d'un médicament choisi dans le groupe constitué par la fluoxétine, la paroxétine, la sertraline, la cimétidine, l'amiodarone, la doxépine, la ticlopidine et l'halopéridol par ladite patiente atteinte d'un cancer du sein,
dans lequel la présence dudit rapport d'expression HOXB13 : IL17BR inférieur à -1,339, l'absence dudit génotype et la non prise dudit médicament indiquent que ladite patiente est susceptible de survivre plus longtemps qu'une patiente atteinte d'un cancer du sein comparable qui présente un tissu cancéreux avec un rapport d'expression HOXB13 : IL17BR supérieur à -1,339, qui présente ledit génotype et qui prend ledit médicament.

6. Procédé selon la revendication 5, dans lequel ladite patiente atteinte d'un cancer du sein est une patiente atteinte d'un cancer du sein sans envahissement ganglionnaire.

7. Procédé selon la revendication 5, dans lequel l'absence dudit rapport d'expression HOXB13 : IL17BR inférieur à -1,339, la présence dudit génotype et la prise dudit médicament indiquent que ladite patiente est susceptible de survivre moins longtemps qu'une patiente atteinte d'un cancer du sein comparable qui présente un tissu cancéreux avec un rapport d'expression HOXB13 : IL17BR inférieur à -1,339, qui ne présente pas ledit génotype et qui ne prend pas ledit médicament.

8. Procédé selon la revendication 5, dans lequel la détermination de la présence ou non dudit rapport d'expression HOXB13 : IL17BR inférieur à -1,339 dans un tissu cancéreux de ladite patiente atteinte d'un cancer du sein comprend l'utilisation de la méthode de calcul HOXB13 : IL-17BR.

9. Procédé d'évaluation de la probabilité d'une récidive de cancer, dans lequel ledit procédé comprend :
(a) la détermination de la présence ou non d'un rapport d'expression HOXB13 : IL17BR supérieur à -1,339 dans un tissu cancéreux d'une patiente atteinte d'un cancer du sein prenant du tamoxifène, et
(b) la détermination de la prise ou non d'un médicament choisi dans le groupe constitué par la fluoxétine, la paroxétine, la sertraline, la cimétidine, l'amiodarone, la doxépine, la ticlopidine et l'halopéridol par ladite patiente atteinte d'un cancer du sein, dans lequel ledit médicament est pris en même temps que ledit tamoxifène, dans lequel la présence dudit rapport d'expression HOXB13 : IL17BR supérieur à -1,339 et la prise dudit médicament indiquent que ladite patiente est susceptible de subir une récidive de cancer.

10. Procédé selon la revendication 9, dans lequel ladite patiente atteinte d'un cancer du sein est une patiente atteinte d'un cancer du sein sans envahissement ganglionnaire.

11. Procédé selon la revendication 9, dans lequel l'absence dudit rapport d'expression HOXB13 : IL17BR supérieur à -1,339 et la non prise dudit médicament indiquent que ladite patiente est susceptible de survivre sans récidive ou de survivre sans maladie.

12. Procédé selon la revendication 9, dans lequel la détermination de la présence ou non dudit rapport d'expression HOXB13 : IL17BR supérieur à -1,339 dans un tissu cancéreux de ladite patiente atteinte d'un cancer du sein prenant du tamoxifène comprend l'utilisation de la méthode de calcul HOXB13 : IL-17BR.
